(19) Europäisches Patentamt
European Patent Office
Office européen des brevets

(11) Veröffentlichungsnummer: **0 315 071 B1**

(12) # EUROPÄISCHE PATENTSCHRIFT

(45) Veröffentlichungstag der Patentschrift: **20.07.94**

(51) Int. Cl.[5]: **C07C 63/66**, C07C 65/19, C07C 63/74, C07C 49/567, C07C 69/76

(21) Anmeldenummer: **88118011.1**

(22) Anmeldetag: **28.10.88**

Die Akte enthält technische Angaben, die nach dem Eingang der Anmeldung eingereicht wurden und die nicht in dieser Patentschrift enthalten sind.

(54) **Neue Benzocyclohepten-Derivate, deren Herstellung und Verwendung für Pharmazeutika.**

(30) Priorität: **06.11.87 CH 4346/87**
**10.08.88 CH 3018/88**

(43) Veröffentlichungstag der Anmeldung:
**10.05.89 Patentblatt 89/19**

(45) Bekanntmachung des Hinweises auf die Patenterteilung:
**20.07.94 Patentblatt 94/29**

(84) Benannte Vertragsstaaten:
**AT BE CH DE ES FR GB GR IT LI LU NL SE**

(56) Entgegenhaltungen:
**EP-A- 0 002 742**
**EP-A- 0 084 667**
**EP-A- 0 176 032**

(73) Patentinhaber: **F. HOFFMANN-LA ROCHE AG**
**Postfach 3255**
**CH-4002 Basel(CH)**

(72) Erfinder: **Klaus, Michael, Dr.**
**Am Hellenrain 6**
**D-7858 Weil/Rhein(DE)**
Erfinder: **Mohr, Peter, Dr.**
**Martinsgasse 9**
**CH-4051 Basel(CH)**

(74) Vertreter: **Lederer, Franz, Dr. et al**
**Lederer, Keller & Riederer**
**Patentanwälte**
**Prinzregentenstrasse 16**
**D-80538 München (DE)**

EP 0 315 071 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Benzocycloheptenderivate, ihre Herstellung und pharmazeutische Präparate, die diese Verbindungen enthalten. Die erfindungsgemässen Benzocycloheptenderivate sind Verbindungen der allgemeinen Formel

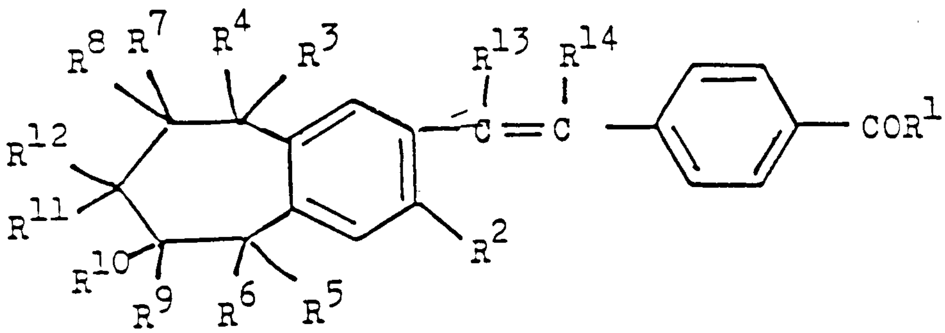

worin

| | |
|---|---|
| $R^1$ | Hydroxy, nieder-Alkoxy, Amino, mono- oder di-nieder-Alkylamino; |
| $R^2$ | Wasserstoff, Alkyl, Alkoxy oder Halogen; |
| $R^3$, $R^4$, $R^5$, $R^6$, $R^{11}$ und $R^{12}$ | unabhängig voneinander Wasserstoff oder nieder-Alkyl; |
| $R^3$ und $R^5$ | gemeinsam eine Methylen- oder Hydroxymethylengruppe; |
| $R^7$, $R^8$, $R^9$ und $R^{10}$ | Wasserstoff oder nieder-Alkyl; oder |
| $R^{11}$ und $R^{12}$ | gemeinsam eine Oxogruppe oder Spiro-cyclo-niederalkyl; oder $R^{11}$ Wasserstoff und $R^{12}$ Hydroxy oder Acetoxy; und einer der Reste $R^{13}$ und $R^{14}$ Wasserstoff und der andere nieder-Alkyl oder Trifluormethyl bedeutet, |

sowie physiologisch verträgliche Salze von Carbonsäuren der Formel I.

Eine bevorzugte Gruppe der erfindungsgemässen Verbindungen sind solche, worin $R^1$ Hydroxy, nieder-Alkoxy, Amino, mono- oder di-nieder-Alkylamino; $R^2$ Wasserstoff, Alkyl, Alkoxy oder Halogen; $R^3$, $R^4$, $R^5$, $R^6$, $R^{11}$ und $R^{12}$ unabhängig voneinander Wasserstoff oder nieder-Alkyl; $R^3$ und $R^5$ gemeinsam eine Methylengruppe; $R^7$, $R^8$, $R^9$ und $R^{10}$ Wasserstoff oder nieder-Alkyl; $R^{11}$ und $R^{12}$ gemeinsam eine Oxogruppe, oder $R^{11}$ Wasserstoff und $R^{12}$ Hydroxy; und einer der Reste $R^{13}$ und $R^{14}$ Wasserstoff und der andere nieder-Alkyl oder Trifluormethyl bedeutet, sowie physiologisch verträgliche Salze von Carbonsäuren der Formel I. Weiterhin bevorzugt sind Verbindungen der Formel I, in denen $R^1$ hydroxy oder nieder-Alkoxy ist und solche, in denen $R^2$ Wasserstoff, und $R^3$-$R^{12}$ Wasser- stoff oder nieder-Alkyl sind.

Andere bevorzugte Verbindungen der Formel I sind solche, in deren mindestens einer der Reste $R^3$-$R^{12}$ nieder-Alkyl ist; solche, in denen $R^{11}$ und $R^{12}$ gemeinsam eine Oxogruppe darstellen; solche, in denen $R^{11}$ Wasserstoff und $R^{12}$ Hydroxy bedeuten; und solche, in denen $R^3$ und $R^5$ gemeinsam eine Methylengruppe darstellen. Von besonderem Interesse ist das Aethyl p-[(E)-2-(6,7,8,9-tetrahydro-7,7-dimethyl-5H-benzocyclohepten-2-yl)propenyl]benzoat.

Die Bezeichnung "nieder" bezieht sich auf Gruppen mit 1-6 C-Atomen. Alkyl- und Alkoxygruppen können geradkettig oder verzweigt sein, wie Methyl, Aethyl, Propyl, Isopropyl, Butyl, sek.-Butyl bzw. Methoxy, Aethoxy, Propoxy, Isopropoxy, Butoxy und sek.-Butoxy. Alkyl- und Alkoxygruppen $R^2$ enthalten vorzugsweise bis zu 10 C-Atome, wie Methyl, Aethyl, Propyl, Butyl, Octyl, Nonyl, Decyl bzw. Methoxy, Aethoxy, Propoxy, Butoxy, Octyloxy, Nonyloxy und Decyloxy. Halogen umfasst Fluor, Chlor, Brom und Jod.

Beispiele physiologischer verträglicher Salze von Carbonsäuren der Formel I sind Alkalisalze, wie Na- und K-Salze, Erdalkalimetallsalze wie Ca-Salze und substituierte und unsubstituiert Ammoniumsalze.

Die Verbindungen der Formel I können als trans- oder cis-Isomere oder cis/trans-Isomerengemische vorliegen. Im allgemeinen sind die trans-Verbindungen der Formel I bevorzugt.

Die Verbindungen der Formel I und ihre Salze können erfindungsgemäss dadurch hergestellt werden, dass man eine Verbindung der allgemeinen Formel

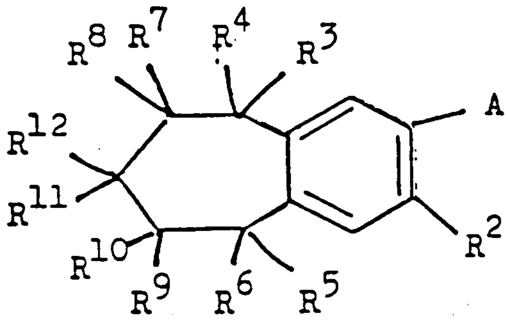

II

mit einer Verbindung der allgemeinen Formel

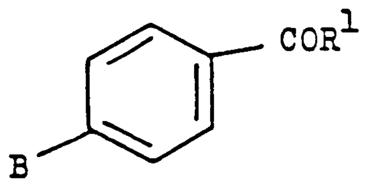

III

umsetzt,
wobei A einen Rest $-CH(R^{13})P^{+}(Q)_3Y^{-}$ und B einen Rest $-CO(R^{14})$-; oder A einen Rest $-CO(R^{13})$- und B einen Rest $-CH_2-PO(OAlk)_2$; Q Aryl, $Y^{-}$ das Anion einer organischen oder anorganischen Säure, Alk eine niedere Alkylgruppe darstellt und $R^1$-$R^{14}$ die oben angegebene Bedeutung haben und wobei eine als $R^{12}$ oder $R^{11}$ und $R^{12}$ anwesende Hydroxy- bzw. Oxogruppe in geschützter Form vorliegt, aus dem Kondensationsprodukt eine gegebenenfalls anwesende Hydroxy- oder Oxoschutzgruppe abspaltet und gewünschtenfalls eine Estergruppe $-COR^1$ der so erhaltenen Verbindung der Formel I verseift oder in eine Carboxaminogruppe oder eine N-mono- oder dialkylierte Carboxaminogruppe überführt.

Die Umsetzung der Verbindungen der Formeln II und III kann nach den bekannten Methoden der Wittig- oder Horner-Reaktion durchgeführt werden.

Bei der Wittig-Reaktion, d.h., bei Verwendung einer Verbindung der Formel II mit A = $-CH(R^{13})P^{+}(Q)_3Y^{-}$ oder der Formel III mit B = $-CH(R^{14})P^{+}(Q)_3Y^{-}$, werden die Komponenten in Gegenwart eines säurebindenden Mittels, z.B. in Gegenwart einer starken Base, wie z.B. Butyllithium, Natriumhydrid oder dem Natriumsalz von Dimethylsulfoxyd, vornehmlich aber in Gegenwart eines gegebenenfalls durch niederes Alkyl substituierten Aethylenoxyds wie 1,2-Butylenoxyd, gegebenenfalls in einem Lösungsmittel, z.B. in einem Aether, wie Diäthyläther oder Tetrahydrofuran, oder in einem aromatischen Kohlenwasserstoff, wie Benzol in einem zwischen der Raumtemperatur und dem Siedepunkt des Reaktionsgemisches liegenden Temperaturbereich miteinander umgesetzt.

Von den anorganischen Säureanionen $Y^{-}$ ist das Chlor-und Brom-ion oder das Hydrosulfat-ion, von den organischen Säureanionen ist das Tosyloxy-ion bevorzugt. Der Arylrest Q ist vorzugsweise ein Phenylrest oder ein substituierter Phenylrest wie p-Tolyl.

Bei der Horner-Reaktion, d.h. bei Verwendung einer Verbindung der Formel II mit A = $-CH(R^{13})-P(O)-(OAlk)_2$ oder der Formel III mit B = $-CH(R^{14})-P(O)(OAlk)_2$ werden die Komponenten mit Hilfe einer Base und vorzugsweise in Gegenwart eines inerten organischen Lösungsmittels, z.B. mit Hilfe von Natriumhydrid in Benzol, Toluol, Dimethylformamid, DMSO, Tetrahydrofuran, Dioxan oder 1,2-Dimethoxyäthan, oder auch mit Hilfe eines Alkoholates in einem Alkanol, z.B. Kalium-t-butylat in t-Butanol oder Natriummethylat in Methanol, in einem zwischen 0° und dem Siedepunkt des Reaktionsgemisches liegenden Temperaturbereich kondensiert.

Die Alkoxyreste OAlk sind vornehmlich niedere Alkoxyreste mit 1-6 Kohlenstoffatomen, wie Methoxy, Aethoxy oder Propoxy.

Ein Carbonsäureester der Formel I kann in an sich bekannter Weise, z.B. durch Behandeln mit Alkalien, insbesondere durch Behandeln mit wässriger alkoholischer Natron-oder Kalilauge in einem zwischen Raumtemperatur und dem Siedepunkt des Reaktionsgemisches liegenden Temperaturbereich zu einer Carbonsäure der Formel I hydrolysiert werden.

Eine Carbonsäure der Formel I kann in an sich bekannter Weise, z.B. durch Behandeln mit Thionylchlorid, vorzugsweise in Pyridin, oder Phosphortrichlorid in Toluol oder Oxalylchlorid in Dimethylformamid/Benzol in das Säurechlorid übergeführt werden, das durch Umsetzen mit Alkoholen in Ester, mit

Aminen in das entsprechende Amid umgewandelt werden kann. Amide können auch aus Carbonsäureestern der Formel I z.B. durch Behandeln mit Lithiumamid direkt erhalten werden. Das Lithiumamid wird vorteilhaft bei Raumtemperatur mit dem betreffenden Ester zur Reaktion gebracht. Carbonsäureamide der Formel I können in an sich bekannter Weise mono- oder di-alkyliert werden, z.B. durch Behandlung mit Alkylhalogeniden in Gegenwart einer Base, wobei diese Reaktion bei der Herstellung von Verbindungen der Formel I, in denen $R^{11}$ und $R^{12}$ eine Oxogruppe darstellt, zweckmässig vor der Abspaltung der Oxoschutzgruppe vorgenommen wird.

Die Verbindungen der Formel I können in trans- oder cis-Form vorliegen. Bei der Herstellung fallen sie mehrheitlich in der trans-Form an. Gegebenenfalls anfallende cis-Anteile können in an sich bekannter Weise, falls erwünscht, abgetrennt oder, z.B. durch Säurekatalyse oder photochemisch zu den trans-Isomeren isomerisiert werden.

Die Ausgangsverbindungen der Formeln II und III können, soweit ihre Herstellung nicht bekannt oder nachstehend beschrieben ist, in Analogie zu bekannten oder den nachstehend beschriebenen Methoden hergestellt werden.

Eine Verbindung der Formel

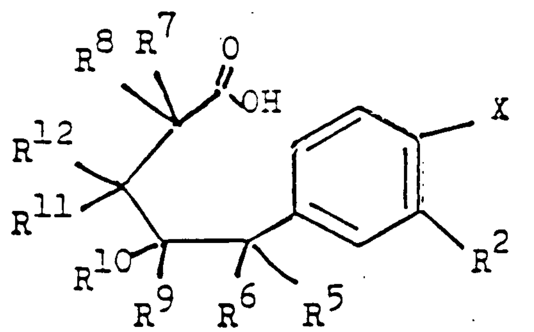

IV

worin $R^2$ und $R^5$-$R^{12}$ die früher angegebene Bedeutung haben, und X Wasserstoff oder Brom ist, kann durch Behandlung mit Säuren wie Polyphosphorsäure oder durch Ueberführung in das Säurechlorid und nachfolgende Behandlung mit einem Friedel-Crafts-Katalysator zu einer Verbindung der Formel

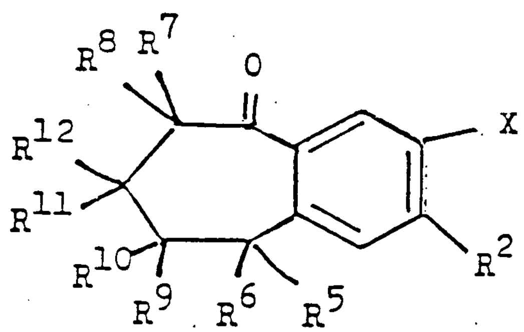

V

cyclisiert werden. Die Oxogruppe in der Verbindung der Formel V kann dann durch Behandlung mit Reduktionsmitteln entfernt werden, wobei man eine der Formel II entsprechende Verbindung erhält, in der A Wasserstoff oder ein Bromatom darstellt. Die Bromverbindung kann nach Ueberführung in eine Grignard-Verbindung mit einem Aldehyd $R^{13}$-CHO in den entsprechenden Benzylalkohol der Formel

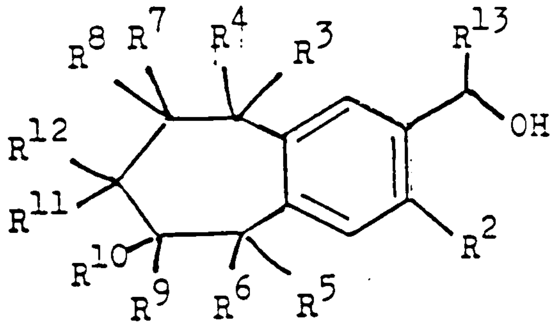

X

umgewandelt werden. Die Verbindung der Formel X kann dann über das entsprechende Benzylhalogenid in ein Phosphoniumsalz, d.h. eine Verbindung der Formel II in der A einen Rest $-CH(R^{13})P^{+}(Q)_3Y^{-}$ darstellt, übergeführt werden.
Andererseits kann die Verbindung der Formel X mittels Oxidationsmitteln wie Mangandioxid zum Keton, d.H. einer Verbindung der Formel II mit A = $-CO(R^{13})$ oxidiert werden. Aus Verbindungen der Formel V mit X= Wasserstoff erhaltene Reduktionsprodukte können mit einem Säurechlorid $R^{13}$ COCl in einer Friedel-Crafts-Reaktion zu Verbindungen der Formel II umgesetzt werden, in denen A ein Rest $-CO(R^{13})$ ist.

Die Verbindungen der Formel IV können dadurch hergestellt werden, dass man Glutarsäureanhydrid oder ein entsprechend substituiertes Derivat davon nach Friedel-Crafts mit Benzol bzw. Brombenzol oder einem o-Alkyl, o-Alkoxy-oder o-Halogenderivat davon zu einer Verbindung der Formel

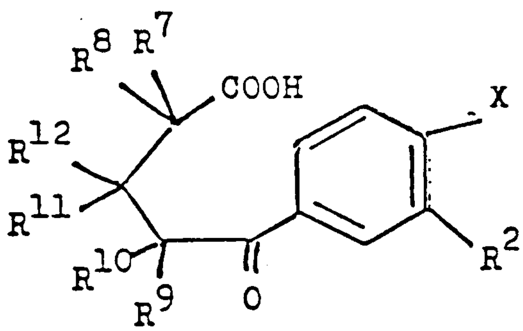

VI

umsetzt, und die Oxogruppe in der Verbindung der Formel VI durch einen Substituenten $R^5$ und/oder $R^6$ ersetzt.

Eine Verbindung der Formel

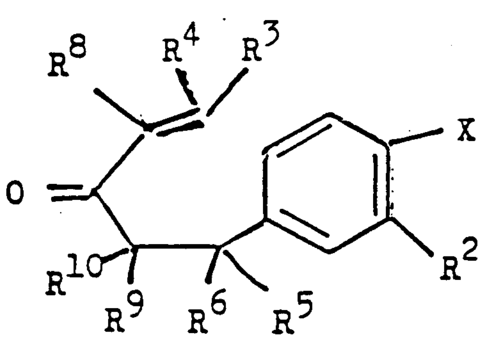

VII

kann durch Behandlung mit einem Friedel-Crafts-Katalysator wie Aluminiumchlorid zu einer Verbindung der Formel VIII, cyclisiert werden. Die Verbindungen der Formel VII können durch Friedel-Crafts-Reaktion von Verbindungen der Formel

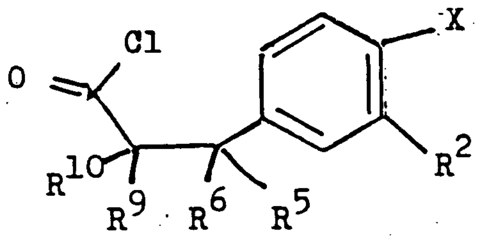

IX

mit Verbindungen der Formel $R^8$-CH = C($R^3$,$R^4$) erhalten werden.

Die vorstehend skizzierten und weitere Herstellungsweisen für die Verbindungen der Formel II werden in den Beispielen ausführlicher beschrieben.

Die Verbindungen der Formel I sind therapeutisch wirksam. Sie besitzen insbesondere anti-seborrhoische, anti-keratinisierende, anti-neoplastische und anti-allergische/anti-inflammatorische Wirksamkeit. Die Erfindung betrifft somit auch die Verbindungen der Formel I und deren Salze zur Verwendung bei der Herstellung von Pharmazeutika mit einem solchen Wirkungsspektrum.

In dem Dokument EP-A-0 084 667 verden Verbindungen der Formel I mit ahnlichem wirkungspektrum beschrieben, in denen der nicht-aromatische Ring jedoch nicht als 7-Ring definiert ist.

In der in Europ. J. Cancer 10, 731-7 (1974) beschriebenen Versuchsanordnung zur Behandlung chemisch induzierter Hauptpapillome bei Mäusen wurden beispielsweise die in Tabelle I angeführten Aktivitäten ermittelt.

Tabelle I

| Verbindung von Beispiel | Dosis pro 14 Tage [mg/kg] 4 x | Regression der Papillomdurchmesser [%] | A-Hypervitaminose Dosis pro 14 Tage [mg/kg] 10 x |
|---|---|---|---|
| 1 | 3<br>1,5<br>0,75 | 59<br>57<br>25 | 3 |
| 3 | 0,75<br>0,4<br>0,2 | 67<br>56<br>28 | 0,75 |
| 4 | 0,2<br>0,1 | 66<br>47 | 0,1 |
| 5 | 0,1 | 54 | 0,1 |
| 8 | 50<br>25<br>12,5 | 78<br>70<br>43 | 50 |

Die Verbindungen der Formel I können zur topischen und systemischen Therapie von benignen und malignen Neoplasien, von prämalignen Läsionen, sowie ferner auch zur systemischen und topischen Prophylaxe der genannten Affektionen verwendet werden.

Sie sind des weiteren für die topische und systemische Therapie von Akne, Psoriasis und anderen mit einer verstärkten oder pathologisch veränderten Verhornung einhergehenden Dermatosen, wie auch von entzündlichen und allergischen dermatologischen Affektionen geeignet. Die Verfahrensprodukte der Formel I können ferner auch zur Bekämpfung von Schleimhauterkrankungen mit entzündlichen oder degenerativen bzw. metaplastischen Veränderungen eingesetzt werden.

Die Mittel können enteral, parenteral oder topisch verabreicht werden. Für die enterale Applikation eignen sich z.b. Mittel in Form von Tabletten, Kapseln, Dragées, Sirupen, Suspensionen, Lösungen und Suppositorien. Für die parenterale Applikation sind Mittel in Form von Infusions-oder Injektionslösungen geeignet.

Die Dosierungen, in denen die Präparate verabreicht werden, können je nach Anwendungsart und Anwendungsweg sowie nach den Bedürfnissen der Patienten variieren. Im allgemeinen kommen für den Erwachsenen tägliche Dosen von etwa 0,1-50 mg/kg, vorzugsweise 1-15 mg/kg in Betracht.

Die Präparate können in einer oder mehreren Dosierungen verabreicht werden. Eine bevorzugte Darreichungsform sind Kapseln mit einem Gehalt von ca. 5-200 mg Wirkstoff.

Die Präparate können inerte oder auch pharmakodynamisch aktive Zusätze enthalten. Tabletten oder Granula z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze, sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dgl. bestehen. Voraussetzung ist, dass alle bei der Herstellung der Präparate verwendeten Hilfsstoffe untoxisch sind.

Zur topischen Anwendung werden die Wirkstoffe zweckmässig in Form von Salben, Tinkturen, Crèmen, Lösungen, Lotionen, Sprays, Suspensionen und dgl. verwendet. Bevorzugt sind Salben und Crèmen sowie Lösungen. Diese zur topischen Anwendung bestimmten Präparate können dadurch hergestellt werden, dass man die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig ca. 0,1-5%ige, vorzugsweise 0,3-2%ige Lösungen, sowie ca. 0,1-5%ige, vorzugsweise ca. 0,3-2%ige Salben oder Crèmen geeignet.

Den Präparaten kann gegebenenfalls ein Antioxydationsmittel, z.B. Tocopherol, N-Methyl-γ-tocopheramin sowie butyliertes Hydroxyanisol oder butyliertes Hydroxytoluol beigemischt sein.

Die nachstehenden Beispiele erläutern die Erfindung weiter. Die Temperaturen sind in Celsiusgraden angegeben.

Beispiel 1

A. Herstellung des Ausgangsmaterials:

a) Zu 30 g p-Bromhydrozimtsäure gibt man 19,0 ml Thionylchlorid und erhitzt 30 Minuten zum Rückfluss. Nach Abkühlen wird das überschüssige Reagens durch Abdampfen entfernt (zuletzt bei 0,5 Torr) und das rohe Säurechlorid in 300 ml Schwefelkohlenstoff gelöst. Nach Abkühlen auf -10° werden unter Argonatmosphäre 3,5 ml Zinntetrachlorid zugegeben. Danach werden 25 ml Isobutylen eingeleitet und das Reaktionsgemisch wird langsam auf Raumtemperatur aufgewärmt. Nach erneutem Abkühlen auf 0° werden 35 g Aluminiumtrichlorid zugegeben. Das Reaktionsgemisch wird über Nacht gerührt, auf Eis gegossen, mit Diäthyläther extrahiert und der Extrakt mit Wasser und Phosphatpuffer (pH 7) gewaschen und getrocknet. Nach Eindampfen wird der Rückstand an Silicagel mit Petroläther (tiefsiedend) und Aethylacetat (9:1) chromatographiert. Man erhält 2-Brom-5,6,8,9-tetrahydro-9,9-dimethyl-7-benzocycloheptenon in Form gelblicher Kristalle vom Smp. 67-68° (aus Hexan).

b) 12,4 g der wie vorstehend erhaltenen Verbindung werden in 60 ml Diäthylenglykol gelöst und mit 6,9 ml Hydrazinhydrat und 8,9 g Kaliumhydroxid versetzt. Das Reaktionsgemisch wird auf 195° erwärmt und das Verschwinden des Hydrazons gaschromatographisch verfolgt. Nach ca. 12 Stunden wird das Reaktionsgemisch abgekühlt, mit Wasser versetzt und mit tiefsiedendem Petroläther extrahiert. Der Extrakt wird mit Wasser gewaschen, getrocknet und eingedampft. Nach Chromatographie an Silicagel mit Petroläther (tiefsiedend) wird 2-Brom-6,7,8,9-tetrahydro-9,9-dimethyl-5H-benzocyclohepten als farbloses Oel erhalten.

c) Dieses Oel wird mit 675 mg Mg-Spänen unter Argon in 30 ml absolutem Tetrahydrofuran in das Grignard-Reagens übergeführt. Nach Abkühlen auf -10° wird Acetaldehyd in grossem Ueberschuss zugesetzt, das Reaktionsgemisch danach mit gesättigter Ammoniumchloridlösung hydrolysiert, mit Diäthyläther extrahiert und der Extrakt mit Wasser gewaschen und getrocknet. Nach Chromatographie an Silicagel mit Petroläther (tiefsiedend) und Aethylacetat (9:1) wird 6,7,8,9-Tetrahydro-α,9,9-trimethyl-5H-benzocyclohepten-2-methanol als farbloses Oel erhalten.

B. Wittig-Reaktion:

783 mg 6,7,8,9-Tetrahydro-α,9,9-trimethyl-5H-benzocyclohepten -2-methanol werden in 8 ml Acetonitril gelöst, mit 1,77 g Triphenylphosphinhydrobromid versetzt und 24 Stunden bei 40° gerührt. Danach wird das Lösungsmittel unter vermindertem Druck abgedampft und der Rückstand zwischen Hexan und

Aethanol/Wasser (8:2) verteilt. Die schwerere Phase wird eingedampft und zweimal mit Methylenchlorid abgedampft. Nach Trocknen unter vermindertem Druck erhält man 1,91 g Phosphoniumsalz, das als Rohprodukt weiterverarbeitet wird.

Das Phosphoniumsalz wird mit 910 mg 4-Formylbenzoesäureäthylester in 5 ml Butylenoxid 20 Stunden zum Rückfluss erwärmt. Nach Abkühlen wird das Reaktionsgemisch zwischen Aethanol/Wasser (8:2) und Hexan verteilt, die leichtere Phase getrocknet und eingedampft. Nach Chromatographie an Silicagel mit Petroläther (tiefsiedend) und Aethylacetat (97:3) werden 820 mg Aethyl p-[(E)-2-(6,7,8,9-tetrahydro-9,9-dimethyl-5H-benzocyclohepten -2-yl)propenyl]benzoat vom Smp. 69-70° (aus Hexan) erhalten.

Beispiel 2

In Analogie zu Beispiel 1B erhält man aus 6,7,8,9-Tetrahydro-α,5,5-trimethyl-5H-benzocyclohepten-2-methanol (hergestellt in Analogie zu Beispiel 1A aus 2-Brom-5,6,8,9-tetrahydro-5,5-dimethyl-7-benzocycloheptenon) das Aethyl p-[(E)-2-(6,7,8,9-tetrahydro-5,5-dimethyl-5H-benzocyclohepten-2-yl)propenyl]benzoat vom Smp. 48-50°.

Beispiel 3

In Analogie zu Beispiel 1B erhält man aus 6,7,8,9-Tetrahydro-α,5,9,9-tetramethyl-5H-benzocyclohepten-2-methanol (hergestellt aus 2-Brom-5,6,8,9-tetrahydro-5,9,9-trimethyl-7-benzocycloheptenon in Analogie zu Beispiel 1A) das Aethyl p-[(E)-2-(6,7,8,9-tetrahydro-5,9,9-trimethyl-5H -benzocyclohepten-2-yl)propenyl]-benzoat vom Smp. 77-78°.

Beispiel 4

In Analogie zu Beispiel 1B erhält man aus 6,7,8,9-Tetrahydro-α,5,5,9-tetramethyl-5H-benzocyclohepten-2-methanol (hergestellt aus 2-Brom-5,6,8,9-tetrahydro-5,5,9-trimethyl-7-benzocycloheptenon in Analogie zu Beispiel 1A) das Aethyl p-[(E)-2-(6,7,8,9-tetrahydro-5,5,9-trimethyl-5H -benzocyclohepten-2-yl)propenyl]-benzoat vom Smp. 43-46°.

Beispiel 5

In Analogie zu Beispiel 1B erhält man aus 6,7,8,9-Tetrahydro-α,5,5,9,9-pentamethyl-5H -benzocyclohepten-2-methanol das Aethyl p-[(E)-2-(6,7,8,9-tetrahydro-5,5,9,9-tetramethyl-5H -benzocyclohepten-2-yl)-propenyl]benzoat vom Smp. 103-104°.

Das Ausgangsmaterial kann wie folgt hergestellt werden:

47,1 g Brombenzol und 37,8 g Phoron werden in Gegenwart von 40 g Aluminiumtrichlorid in 600 ml Schwefelkohlenstoff 48 Stunden bei Raumtemperatur gerührt. Das Reaktionsgemisch wird dann auf Eis gegossen, mit Diäthyläther extrahiert, der Extrakt mit Wasser gewaschen, getrocknet und eingedampft. Nach Chromatographie an Silicagel mit Petroläther (tiefsiedend) und Aethylacetat (9:1) und Umkristallisation aus Hexan erhält man 2-Brom-5,6,8,9-tetrahydro-5,5,9,9-tetramethyl-7H -benzocyclohepten-7-on vom Smp. 104-105°.

Die letztgenannte Verbindung kann in Analogie zu Beispiel 1A Absatz b) weiter zu 6,7,8,9-Tetrahydro-α,5,5, 9,9-pentamethyl-5H -benzocyclohepten-2-methanol umgesetzt werden.

Beispiel 6

In Analogie zu Beispiel 1B erhält man aus 6,7,8,9-Tetrahydro-α,7,7,9-tetramethyl-5H-benzocyclohepten-2-methanol das Aethyl p-[(E)-2-(6,7,8,9-tetrahydro-7,7,9-trimethyl-5H -benzocyclohepten-2-yl)propenyl]-benzoat vom Smp. 68-71°.

Das Ausgangsmaterial kann wie folgt hergestellt werden:

Zu einer Lösung 4,28 g 3-Brom-6,7,8,9-tetrahydro-7,7-dimethyl-5H -benzocyclohepten-5-on in 20 ml absolutem Diäthyläther werden bei Raumtemperatur langsam 15 ml ca. 1,5M frisch hergestellte Methylmagnesiumjodidlösung in Diäthyläther zugetropft. Das Reaktionsgemisch wird 30 Minuten gerührt, danach mit gesättigter Ammoniumchloridlösung hydrolysiert, mit Diäthyläther extrahiert und der Extrakt mit Wasser gewaschen und getrocknet und eingedampft. Man erhält 4,8 g eines farblosen Oels, das mit 80 ml 37%iger Salzsäure versetzt werden und intensiv über Nacht gerührt wird. Danach wird mit Diäthyläther extrahiert, der Extrakt mit Wasser gewaschen, getrocknet und eingedampft. Nach Filtrieren über Silicagel mit

tiefsiedendem Petroläther erhält man 3,82 g eines farblosen Oels, das gemäss gaschromatographischer Analyse aus einem 2:1-Gemisch von exo- und endocyclischem Olefin besteht. 3,30 g dieses Oels werden unter Argon in 10 ml absolutem Tetrahydrofuran mit 330 mg Magnesiumspänen umgesetzt und das Grignard-Reagens bei -10° mit einem grossen Ueberschuss an Acetaldehyd reagieren gelassen. Nach 15 Minuten wird das Reaktionsgemisch mit gesättigter Ammoniumchloridlösung hydrolysiert, mit Diäthyläther extrahiert, der Extrakt mit Wasser gewaschen, getrocknet und eingedampft. Nach Chromatographie an Silicagel mit Petroläther (tiefsiedend) und Aethylacetat (8:2) erhält man 2,15 g sek. Alkohol als farbloses Oel (Gemisch von endo- und exocyclischem Olefin). Dieses Oel wird in 20 ml absolutem Aethanol über 150 mg Palladium-Kohle bei Normaldruck hydriert. Nach Aufnahme eines Aequivalents Wasserstoff wird der Katalysator abfiltriert, das Reaktionsgemisch eingedampft und an Silicagel mit Petroläther (tiefsiedend) und Aethylacetat (8:2) chromatographiert. Man erhält 1,17 g 6,7,8,9-Tetrahydro-α,7,7,9-tetramethyl-5H-benzocyclohepten-2-methanol als 1:1-Diastereomerengemisch in Form eines farblosen Oels.

Beispiel 7

In Analogie zu Beispiel 1B erhält man aus 6,7,8,9-Tetrahydro-α,7,7,9,9-pentamethyl-5H-benzocyclohepten-2-methanol das Methyl p-[(E)-2-(6,7,8,9-tetrahydro-7,7,9,9-tetramethyl-5H -benzocyclohepten-2-yl)-propenyl]benzoat vom Smp. 88-89°.

Das Ausgangsmaterial kann wie folgt hergestellt werden:

Eine Lösung von 26,5 g trockenem Methoxymethyltriphenylphosphoniumchlorid in 85 ml abs. Tetrahydrofuran wird bei 0° langsam mit 1,1 Aequivalent 1,55M n-Butyllithium in Hexan versetzt. Nach 30 Minuten werden 13,77 g 3-Brom-6,7,8,9-tetrahydro-7,7-dimethyl-5H -benzocyclohepten-5-on zugesetzt und das Reaktionsgemisch wird ohne Kühlung 1 Stunde gerührt. Das Reaktionsgemisch wird dann auf Eis gegossen, mit Diäthyläther extrahiert, der Extrakt mit Wasser gewaschen, getrocknet und eingedampft. Nach Chromatographie an Silicagel mit Petroläther (tiefsiedend) enthaltend bis zu 5% Aethylacetat erhält man 12,2 g Enoläther als farbloses Oel. (E/Z-Gemisch).

Der Enoläther wird in 820 ml Essigsäure gelöst, die Lösung mit 80 ml 10%iger Schwefelsäure versetzt und über Nacht zum Rückfluss erhitzt. Danach wird das Lösungsmittel zum grössten Teil abgedampft, der Rückstand zwischen Wasser und Diäthyläther verteilt, die ätherische Lösung mit 10%iger Natriumcarbonatlösung gewaschen, getrocknet und eingedampft. Man erhält 11,4 g Aldehyd in Form eines farblosen Oels.

Dieses Oel wird in 70 ml abs. t-Butanol gelöst und unter Argonatmosphäre bei 0° mit 9,1 g Kalium-t-butylat versetzt. Das Reaktionsgemisch wird ohne Kühlung eine halbe Stunde gerührt und dann mit 7,6 ml Methyljodid versetzt. Nach 2 Stunden Rühren bei Raumtemperatur wird auf Eis gegossen, mit Diäthyläther extrahiert, der ätherische Extrakt mit Wasser gewaschen, getrocknet und eingedampft. Chromatographie an Silicagel mit Hexan/Aethylacetat (99,5:0,5) liefert 2,30 g 3-Brom-6,7,8,9-tetrahydro-5,7,7-trimethyl-5H -benzocyclohepten-5-carboxaldehyd als farbloses Oel.

2,30 g des wie vorstehend erhaltenen Aldehyds werden in 12 ml Diäthylenglykol gelöst und mit 0,80 ml Hydrazinhydrat versetzt. Das Reaktionsgemisch wird 3 Stunden auf 100° erwärmt, dann mit 1,1 g Kaliumhydroxid versetzt und 14 Stunden zum Rückfluss erhitzt (Badtemperatur 180°). Nach Abkühlen wird das Reaktionsgemisch auf Eis gegossen, mit Petroläther (tiefsiedend) extrahiert, der Extrakt mit Wasser gewaschen, getrocknet und eingedampft. Nach Filtrieren über Silicagel mit Petroläther (tiefsiedend) erhält man 1,25 g eines farblosen Oels, das unter Argonatmosphäre in 12 ml abs. Tetrahydrofuran mit 140 mg Magnesiumspänen in das Grignard-Reagens übergeführt wird. Nach 1,5 Stunden wird Acetaldehyd bei -10° in grossem Ueberschuss zu dem Grignard-Reagens gegeben und das Reaktionsgemisch 15 Minuten bei Raumtemperatur gerührt. Danach wird das Reaktionsgemisch mit gesättigter Ammoniumchloridlösung hydrolysiert, mit Diäthyläther extrahiert, der Extrakt mit Wasser gewaschen, getrocknet und eingedampft. Nach Chromatographie an Silicagel mit Petroläther (tiefsiedend) und Aethylacetat (8:2) erhält man 760 mg 6,7,8,9-Tetrahydro-α,7,7,9,9-pentamethyl-5H-benzocyclohepten-2-methanol als farbloses Oel.

Beispiel 8

A. Herstellung des Ausgangsmaterials:

a) 82 g 3,3-Dimethyl-5-phenylvaleriansäure werden mit 57 ml Thionylchlorid versetzt und eine halbe Stunde zum Rückfluss erhitzt. Danach wird das überschüssige Reagens abgedampft, zuletzt 1,5 Stunden unter Hochvakuum. Zu 79 g Aluminiumtrichlorid in 650 ml Methylenchlorid werden unter Argonatmosphäre innerhalb von 3,5 Stunden das wie vorstehend hergestellte Säurechlorid, in 300 ml Methylenchlorid gelöst, gegeben. Danach wird noch 10 Minuten gerührt und das Reaktionsgemisch auf Eis gegossen.

Die organische Phase wird mit Wasser gewaschen, getrocknet und eingedampft. Nach Chromatographie an Silicagel mit Petroläther (tiefsiedend)/Aethylacetat (98:2) erhält man 62 g 6,7,8,9-Tetrahydro-7,7-dimethyl-5H-benzocyclohepten-5-on.

b) 34 g der vorstehend erhaltenen Verbindung werden mit 19,5 ml Hydrazinhydrat und 22,4 g Kaliumhydroxid in 200 ml Diäthylenglykol 3 Tage zum Rückfluss erhitzt (Badtemperatur 180-190°). Danach wird das Reaktionsgemisch abgekühlt, zwischen Diäthyläther und Wasser verteilt, die organische Phase mit Wasser gewaschen, getrocknet und eingedampft. Chromatographie an Silicagel mit Petroläther (tiefsiedend) liefert 22,2 g 6,7,8,9-Tetrahydro-7,7-dimethyl-5H-benzocyclohepten als farbloses, beim Stehen kristallisierendes Oel.

c) 6,20 g der vorstehend erhaltenen Verbindung werden unter Argonatmosphäre in 65 ml 1,2-Dichloräthan bei -10° mit 2,9 ml Acetylchlorid und danach mit 6,0 g Aluminiumtrichlorid versetzt. Das Reaktionsgemisch wird aufwärmen gelassen, nach 30 Minuten auf Eis gegossen, mit Diäthyläther extrahiert, der Extrakt mit Wasser gewaschen, getrocknet und eingedampft. Man erhält 8,40 g 2-Acetyl-6,7,8,9-tetrahydro-7,7-dimethyl-5H-benzocyclohepten als farbloses Oel.

B. Horner-Reaktion:

770 mg Natriumhydrid (50% in Mineralöl) werden in 20 ml Dimethylformamid bei Raumtemperatur unter Argonatmosphäre mit 5,30 g Aethyl α-(diäthoxyphosphinyl)-p-toluat versetzt. Nach 2 Stunden Rühren werden 2,55 g 2-Acetyl-6,7,8,9-tetrahydro-7,7-dimethyl-5H-benzocyclohepten, in wenig Dimethylformamid gelöst, zugetropft. Nach 2 Stunden wird das Reaktionsgemisch auf Eis gegossen, mit Diäthyläther extrahiert, der ätherische Extrakt mit Wasser gewaschen, getrocknet und eingedampft. Nach Chromatographie an Silicagel mit Petroläther (tiefsiedend)/Aethylacetat (9:1) erhält man 3,40 g Aethyl p-[(E)-2-(6,7,8,9-tetrahydro-7,7-dimethyl-5H-benzocyclohepten-2-yl)propenyl]benzoat vom Smp. 87-88° (aus Hexan).

<u>Beispiel 9</u>

In Analogie zu Beispiel 8B erhält man aus 2-Acetyl-5,6,8,9-tetrahydrospiro[7H-benzocyclohepten-7,1′-cyclopentan] (hergestellt in Analogie zu Beispiel 8A ausgehend von p-Phenäthyl-cyclopentanessigsäure) das Aethyl p-[(E)-2-[5,6,8,9-tetrahydrospiro[7H-benzocyclohepten-7,1′-cyclopentan]-2-yl]propenyl]benzoat vom Smp. 88-89°.

<u>Beispiel 10</u>

In Analogie zu Beispiel 8B erhält man aus 2-Acetyl-6,7,8,9-tetrahydro-7-methyl-7-äthyl-5H -benzocyclohepten (hergestellt in Analogie zu Beispiel 8A ausgehend von 3-Methyl-3-äthyl-5-phenylvaleriansäure) das Aethyl p-[(E)-2-(7-äthyl-6,7,8,9-tetrahydro-7-methyl-5H -benzocyclohepten-2-yl)propenyl]benzoat vom Smp. 82-83°.

<u>Beispiel 11</u>

In Analogie zu Beispiel 8B erhält man aus 2-Acetyl-6,7,8,9-tetrahydro-7-methyl-5H-benzocyclohepten (hergestellt in Analogie zu Beispiel 8A ausgehend von 3-Methyl-5-phenylvaleriansäure) das Aethyl p-[(E)-2-(6,7,8,9-tetrahydro-7-methyl-5H-benzocyclohepten -2-yl)propenyl]benzoat vom Smp. 40-41°.

<u>Beispiel 12</u>

In Analogie zu Beispiel 8B erhält man aus 2-Acetyl-6,7,8,9-tetrahydro-7,7-äthylendioxy -5H-benzocyclohepten und anschliessende Acetalspaltung das Aethyl p-[(E)-2-(6,7,8,9-tetrahydro-7-oxo-5H-benzocyclohepten -2-yl)propenyl]benzoat vom Smp. 90-91°.

<u>Beispiel 13</u>

In Analogie zu Beispiel 8B erhält man aus 2-Acetyl-5,6,8,9-tetrahydro-6,6,8,8-tetramethyl-7H -benzocyclohepten-7-on das Aethyl p-[(E)-2-(6,7,8,9-tetrahydro-6,6,8,8-tetramethyl-7-oxo -5H-benzocyclohepten-2-yl)propenyl]benzoat vom Smp. 79-80°C.

Das Ausgangsmaterial kann wie folgt hergestellt werden:

Zu 80 mMol Kaliumhydrid (20% in Mineralöl) in 80 ml abs. Tetrahydrofuran werden unter Argonatmosphäre 2,20 g 5,6,8,9-Tetrahydro-7H-benzocyclohepten-7-on gegeben. Nach 15 Minuten wird auf -10° gekühlt und es werden 4,67 ml Methyljodid zugetropft. Das Reaktionsgemisch wird in 1,5 Stunden auf 10° erwärmt und dann unter Eiskühlung und Argonatmosphäre vorsichtig hydrolysiert. Das Reaktionsgemisch wird mit Diäthyläther versetzt, die organische Phase abgetrennt, mit Wasser gewaschen, getrocknet und eingedampft. Das Rohprodukt wird durch Chromatographie an Silicagel gereinigt. Man eluiert zunächst mit Petroläther (tiefsiedend), danach mit Petroläther/Aethylacetat (97,5:2,5), wobei 2,57 g 5,6,8,9-Tetrahydro-6,6,8,8-tetramethyl-7H -benzocyclohepten-7-on als farbloses, beim Stehen kristallisierendes Oel erhalten werden.

620 mg der vorstehend hergestellten Verbindung werden in 10 ml Schwefelkohlenstoff unter Argonatmosphäre bei 0° mit 0,61 ml Acetylchlorid und 1,02 g Aluminiumtrichlorid versetzt. Danach wird das Reaktionsgemisch 3 Stunden bei Raumtemperatur gerührt, auf Eis gegossen, mit Diäthyläther extrahiert und die ätherische Lösung mit 10%iger Natriumcarbonatlösung und Wasser gewaschen, getrocknet und eingedampft. Nach Chromatographie an Silicagel mit Petroläther (tiefsiedend)/Aethylacetat (8:2) erhält man 680 mg 2-Acetyl-5,6,8,9-tetrahydro-6,6,8,8-tetramethyl -7H-benzocyclohepten-7-on.

Beispiel 14

In Analogie zu Beispiel 8B erhält man aus 2-Acetyl-5,6,8,9-tetrahydrospiro[7H-benzocyclohepten -7,1′-cyclopropan] das Aethyl p-[(E)-2-[5,6,8,9-tetrahydrospiro[7H-benzocyclohepten -7,1′-cyclopropan]-2-yl]-propenyl]benzoat vom Smp. 56-58°.

Beispiel 15

In Analogie zu Beispiel 8B erhält man aus 2-Acetyl-6,7,8,9-tetrahydro-5H-benzocyclohepten das Aethyl p-[(E)-2-(6,7,8,9-tetrahydro-5H-benzocyclohepten -2-yl)propenyl]benzoat vom Smp. 53-54°.

Beispiel 16

300 mg Aethyl p-[(E)-2-(6,7,8,9-tetrahydro-5H-benzocyclohepten -2-yl)propenyl]benzoat werden in 20 ml Aethanol mit 2 ml 2,5 M Natriumhydroxidlösung versetzt. Man rührt 48 Stunden bei Raumtemperatur, verdünnt mit Wasser und extrahiert mit Diäthyläther. Die wässrige Phase wird angesäuert, mit Aether extrahiert, die ätherische Phase mit Wasser gewaschen, getrocknet und eingedampft. Man erhält 200 mg p-[(E)-2-(6,7,8,9-Tetrahydro-5H-benzocyclohepten-2-yl)propenyl]benzoesäure vom Smp. 198°.

In analoger Weise können die folgenden Verbindungen erhalten werden:

p-[(E)-2-(6,7,8,9-Tetrahydro-7,7-dimethyl-5H-benzocyclohepten-2-yl)propenyl]benzoesäure, Smp. 192-193°,

p-[(E)-2-(6,7,8,9-Tetrahydro-5,5,9,9-tetramethyl-5H-benzocyclohepten-2-yl)propenyl]benzoesäure, Smp. 221-222°.

Beispiel 17

In Analogie zu Beispiel 8Ac erhält man aus 5,7,8,9-Tetrahydro-6,6,8,8-tetramethyl-5H-benzocyclohepten das 2-Acetyl-6,7,8,9-tetrahydro-6,6,8,8-tetramethyl -5H-benzocyclohepten, daraus in Analogie zu Beispiel 8B das Aethyl p-[(E)-2-(6,7,8,9-tetrahydro-6,6,8,8-tetramethyl -5H-benzocyclohepten-2-yl)propenyl]benzoat und daraus in Analogie zu Beispiel 16 die p-[(E)-2-(6,7,8,9-Tetrahydro-6,6,8,8-tetramethyl -5H-benzocyclohepten-2-yl)propenyl]benzoesäure, Smp. 232-235°C.

Das Ausgangsmaterial kann wie folgt hergestellt werden:

2,57 g 5,6,8,9-Tetrahydro-6,6,8,8-tetramethyl -7H-benzocyclohepten-7-on werden in 50 ml absolutem Diäthyläther gelöst und bei 0°C vorsichtig mit 460 mg Lithiumaluminiumhydrid versetzt. Man entfernt das Kühlbad und rührt noch 2 Stunden bei Raumtemperatur. Dann werden 1,2 g Natriumhydroxid, in wenig Wasser gelöst, portionenweise zugesetzt, worauf das Reaktionsgemisch gerührt wird, bis sich ein filtrierbarer Niederschlag gebildet hat. Nach Abfiltrieren und Eindampfen des Filtrats erhält man 6,7,8,9-Tetrahydro-6,6,8,8-tetramethyl -5H-benzocyclohepten-7-ol als Rohprodukt, das nach Umkristallisieren aus Petroläther (tiefsiedend) bei 66-68°C schmilzt.

1,52 g des vorstehenden Alkohols werden unter Argonatmosphäre in 7 ml Dimethylsulfoxid gelöst und die Lösung wird mit 404 mg Natriumhydrid (50%ig in Mineralöl) versetzt. Das Reaktionsgemisch wird 2 Stunden auf 50°C erwärmt, danach auf 0°C gekühlt und mit 1,18 ml Schwefelkohlenstoff versetzt. Das

Reaktionsgemisch wird 45 Minuten bei Raumtemperatur belassen, danach auf 0°C gekühlt und tropfenweise mit 865 µl Methyljodid versetzt. Nach 45 Minuten Rühren bei Raumtemperatur wird das Reaktionsgemisch auf Eis gegossen, mit Diäthyläther extrahiert, der Extrakt mit Wasser gewaschen und getrocknet und eingedampft. Chromatographie an Silicagel mit Petroläther (tiefsiedend) und Aethylacetat (99:1) liefert S-Methyl-O-(6,7,8,9-tetrahydro-6,6,8,8-tetramethyl -5H-benzocyclohepten-7-yl)dithiocarbonat als gelblichen Feststoff.

1,76 g des vorstehend genannten Dithiocarbonats werden unter Argonatmosphäre in 145 ml Toluol gelöst. Man erhitzt bis zum Siedepunkt und tropft innerhalb einer Stunde 50 ml Toluol zu, in dem 2,67 ml Tributylzinnhydrid und 100 mg Azaisobutyronitril gelöst sind. Nach 30 Minuten kühlt man das Reaktionsgemisch ab, dampft den Hauptteil des Toluols ab und chromatographiert das so erhaltene Rohprodukt an Silicagel. Elution mit Petroläther (tiefsiedend) liefert 6,7,8,9-Tetrahydro-6,6,8,8-tetramethyl-5H-benzocyclohepten.

Beispiel 18

In Analogie zu Beispiel 8B erhält man aus 2-Acetyl-6,7,8,9-tetrahydro-5,9-dimethyl-5,9-methano -5H-benzocyclohepten-10-yl-acetat den p-[(E)-2-(6,7,8,9-Tetrahydro-10-acetoxy-5,9-dimethyl -5,9-methano-5H-benzocyclohepten-2-yl)propenyl]benzoesäure -äthylester und daraus in Analogie zu Beispiel 16 die p-[(E)-2-(6,7,8,9-Tetrahydro-10-hydroxy-5,9-dimethyl -5,9-methano-5H-benzocyclohepten-2-yl)propenyl]benzoesäure, Smp. 188-190°C.

Das Ausgangsmaterial kann wie folgt hergestellt werden:

895 mg 6,7,8,9-Tetrahydro-5,9-dimethyl-5,9-methano-5H-benzocyclohepten-10-ol (Chem. Comm. 1972, 1238) werden unter Argonatmosphäre in 12 ml Dichloräthan bei 0°C mit 0,40 ml Acetylchlorid und 1,30 g Aluminiumtrichlorid versetzt. Das Reaktionsgemisch wird eine halbe Stunde gerührt, auf Eis gegossen, mit Diäthyläther extrahiert, mit Wasser gewaschen und getrocknet. Abdampfen der Lösungsmittel und Chromatographie an Silicagel (tiefsiedender Petroläther/Aethylacetat 9:1) liefert 2-Acetyl-6,7,8,9-tetrahydro-5,9-dimethyl-5,9-methano -5H-benzocyclohepten-10-yl-acetat als farblosen Feststoff.

Beispiel 19

In Analogie zu Beispiel 8B erhält man aus 2-Acetyl-6,7,8,9-tetrahydro-5,9-dimethyl-5,9-methano -5H-benzocyclohepten den p-[(E)-2-(6,7,8,9-Tetrahydro-5,9-dimethyl-5,9-methano -5H-benzocyclohepten-2-yl)-propenyl]benzoesäure-äthylester und daraus in Analogie zu Beispiel 16 die p-[(E)-2-(6,7,8,9-Tetrahydro-5,9-dimethyl-5,9-methano-5H-benzocyclohepten-2-yl)propenyl]benzoesäure vom Smp. 195-200°C.

Das Ausgangsmaterial kann wie folgt hergestellt werden:

6,7,8,9-Tetrahydro-5,9-dimethyl-5,9-methano -5H-benzocyclohepten-10-ol wird mit Natriumhydrid und Dimethylsulfoxid in das Anion überführt und dieses mit Schwefelkohlenstoff zu einem Dithiocarbonat umgesetzt, das mit Methyljodid methyliert wird. Behandlung der so erhaltenen Verbindung mit Tributylzinnhydrid in Gegenwart von Azaisobutyronitril in Toluol liefert das 6,7,8,9-Tetrahydro-5,9-dimethyl-5,9-methano -5H-benzocyclohepten, das mit Acetylchlorid nach Friedel-Crafts, wie in Beispiel 8Ac beschrieben, umgesetzt wird.

Beispiel 20

In Analogie zu Beispiel 1B (1. Abschnitt) erhält man aus 6,7,8,9-Tetrahydro-α,3,7,7-tetramethyl-5H-benzocyclohepten-2-methanol das entsprechende Phosphoniumsalz. 5,30 g davon werden in 35 ml Tetrahydrofuran suspendiert und bei 0° mit 9,0 ml 1,4M nBuLi (Hexan) deprotoniert. Nach 30 Minuten gibt man 2,30 g 4-Formylbenzoesäuremethylester zu und rührt noch 2 Stunden bei Raumtemperatur. Man giesst auf Eiswasser, extrahiert mit $Et_2O$, wäscht mit Wasser, trocknet und dampft ein. Chromatographie an Silicagel (Petroläther tiefsiedend/AcOEt = 93/3), gefolgt von Kristallisation aus n-Hexan, liefert 560 mg Methyl p-[(E)-6,7,8,9-tetrahydro-3,7,7-trimethyl-5H-benzocyclohepten-2-yl)propenyl]benzoat vom Smp. 59-60°.

Das Ausgangsmaterial kann aus o-Bromtoluol und 3,9-Dimethylglutarsäureanhydrid hergestellt werden.

Beispiel 21

In Analogie zu Beispiel 1B erhält man aus 6,7,8,9-Tetrahydro-α,9-dimethyl-5H-benzocyclohepten-2-methanol Aethyl p-[(E)-2-(6,7,8,9-tetrahydro-9-methyl-5H-benzocyclohepten-2-yl)propenyl]benzoat vom Smp. 48-50°.

Das Ausgangsmaterial kann aus Benzosuberon durch die Reaktionssequenz a) Bromierung; b) Addition von MeMgJ; c) Deoxygenierung; d) Metallierung mit Mg und Addition an Acetaldehyd hergestellt werden.

Beispiel 22

In Analogie zu Beispiel 16 erhält man aus dem entsprechenden Methylester (Beispiel 7) p-[(E)-2-(6,7,8,9-Tetrahydro-7,7,9,9-tetramethyl-5H-benzocyclohepten-2-yl)propenyl]benzoesäure vom Smp. 182-183°.

Beispiel 23

In Analogie zu Beispiel 8B und Beispiel 16 erhält man aus 2-Acetyl-6,7,8,9-tetrahydro-7,7-dimethyl-3-octyl-5H-benzocyclohepten p-[(Z)-(6,7,8,9-Tetrahydro-7,7-dimethyl-3-octyl-5H-benzocyclohepten-2-yl)-propenyl]benzoesäure vom Smp. 113-114°.

Das Ausgangsmaterial kann aus Octylbenzol und 3,3-Dimethylgutarsäureanhydrid hergestellt werden.

Beispiel 24

In Analogie zu Beispiel 8B und Beispiel 16 erhält man aus 2-Acetyl-3-brom-6,7,8,9-tetrahydro-7,7-dimethyl-5H-benzocyclohepten p-[(Z)-2-(3-Brom-6,7,8,9-tetrahydro-7,7-dimethyl-5H-benzocyclohepten-2-yl)-propenyl]benzoesäure vom Smp. >270°.

Das Ausgangsmaterial kann aus Brombenzol via folgender Reaktionssequenz hergestellt werden. a) Acylierung mit 3,3-Dimethylglutarsäureanhydrid; 2) Wolf-Kishner-Reduktion; 3) intramolekulare Friedel-Crafts-Acylierung; 4) Wolf-Kishner-Reduktion; 5) Friedel-Crafts-Acetylierung.

Beispiel 25

In Analogie zu Beispiel 16 wird durch Hydrolyse von Z-angereichertem Ester (Mutterlauge von Beispiel 20) p-[(Z)-2-(6,7,8,9-Tetrahydro-3,7,7-trimethyl-5H-benzocyclohepten-2-yl)propenyl]benzoesäure vom Smp. 223-225° erhalten.

Beispiel A

Hartgelatinekapseln können wie folgt hergestellt werden:

| Bestandteile | | mg/Kapsel |
|---|---|---|
| 1. | sprühgetrocknetes Pulver enthaltend 75% Verbindung I | 200 |
| 2. | Natriumdioctylsulfosuccinat | 0,2 |
| 3. | Natriumcarboxymethylcellulose | 4,8 |
| 4. | mikrokristalline Cellulose | 86,0 |
| 5. | Talk | 8,0 |
| 6. | Magnesiumstearat | 1,0 |
| | | Total 300 |

Das sprühgetrocknete Pulver, das auf dem Wirkstoff, Gelatine und mikrokristalliner Cellulose basiert und eine mittlere Korngrösse des Wirkstoffes von <1 µ aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristalliner Cellulose, Talk und Magnesiumstearat vermischt. Das Pulver wird in Kapseln der Grösse 0 abgefüllt.

Beispiel B

Tabletten können wie folgt hergestellt werden:

| Bestandteile | | mg/Tablette |
|---|---|---|
| 1. | Verbindung I als feingemahlenes Pulver | 500 |
| 2. | Milchzucker pulv. | 100 |
| 3. | Masisstärke weiss | 60 |
| 4. | Povidone K30 | 8 |
| 5. | Maisstärke weiss | 112 |
| 6. | Talk | 16 |
| 7. | Magnesiumstearat | 4 |
| | | Total 800 |

Die feingemahlene Substanz wird mit Milchzucker und einem Teil der Maisstärke gemischt. Die Mischung wird mit einer wässrigen Lösung von Povidone K30 befeuchtet und geknetet, und die resultierende Masse granuliert, getrocknet und gesiebt. Das Granulat wird mit der restlichen Maisstärke, Talk und Magnesiumstearat vermischt und zu Tabletten geeigneter Grösse verpresst.

Beispiel C

Weichgelatinekapseln können wie folgt hergestellt werden:

| Bestandteile | | mg/Kapsel |
|---|---|---|
| 1. | Verbindung I | 50 |
| 2. | Triglycerid | 450 |
| | | Total 500 |

10 g Verbindung I werden unter Rühren, Inertbegasung und Lichtschutz in 90 g mittelkettigem Triglycerid gelöst. Diese Lösung wird als Kapselfüllmasse zu Weichgelatinekapseln à 50 mg Wirkstoff verarbeitet.

Beispiel D

Eine Lotion kann wie folgt hergestellt werden:

| Bestandteile | | |
|---|---|---|
| 1. | Verbindung I, feingemahlen | 3,0 g |
| 2. | Carbopol 934 | 0,6 g |
| 3. | Natriumhydroxid | q.s. ad pH 6 |
| 4. | Aethanol, 94% | 50,0 g |
| 5. | entmineralisiertes Wasser | ad 100,0 g |

Der Wirkstoff wird unter Lichtschutz in die Mischung Aethanol, 94%ig/Wasser eingearbeitet. Carbopol 934 wird bis zur vollständigen Gelierung eingerührt und der pH-Wert mit Natriumhydroxid eingestellt.

**Patentansprüche**

**Patentansprüche für folgende Vertragsstaaten : AT, BE, CH, DE, FR, GB, IT, LI, LU, NL, SE**

**1.** Verbindungen der allgemeinen Formel

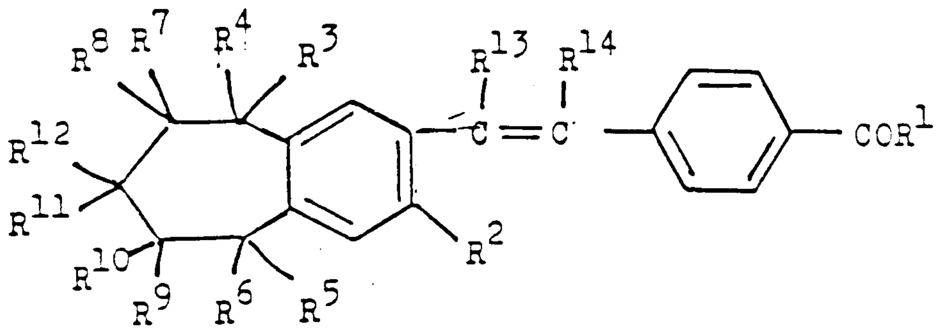

I

worin

| | |
|---|---|
| $R^1$ | Hydroxy, nieder-Alkoxy, Amino, mono- oder di-nieder-Alkylamino; |
| $R^2$ | Wasserstoff, Alkyl, Alkoxy oder Halogen; |
| $R^3$, $R^4$, $R^5$, $R^6$, $R^{11}$ und $R^{12}$ | unabhängig voneinander Wasserstoff oder nieder-Alkyl; |
| $R^3$ und $R^5$ | gemeinsam eine Methylen- oder Hydroxymethylengruppe; |
| $R^7$, $R^8$, $R^9$ und $R^{10}$ | Wasserstoff oder nieder-Alkyl; oder |
| $R^{11}$ und $R^{12}$ | gemeinsam eine Oxogruppe oder Spiro-cyclo-niederalkyl; oder $R^{11}$ Wasserstoff und $R^{12}$ Hydroxy oder Acetoxy; und einer der Reste $R^{13}$ und $R^{14}$ Wasserstoff und der andere nieder-Alkyl oder Trifluormethyl bedeutet, |

sowie physiologisch verträgliche Salze von Carbonsäuren der Formel I.

**2.** Verbindungen gemäss Anspruch 1, worin $R^1$ Hydroxy, nieder-Alkoxy, Amino, mono- oder di-nieder-Alkylamino; $R^2$ Wasserstoff, Alkyl, Alkoxy oder Halogen; $R^3$, $R^4$, $R^5$, $R^6$, $R^{11}$ und $R^{12}$ unabhängig voneinander Wasserstoff oder nieder-Alkyl; $R^3$ und $R^5$ gemeinsam eine Methylengruppe; $R^7$, $R^8$, $R^9$ und $R^{10}$ Wasserstoff oder nieder-Alkyl; $R^{11}$ und $R^{12}$ gemeinsam eine Oxogruppe oder $R^{11}$ Wasserstoff und $R^{12}$ Hydroxy; und einer der Reste $R^{13}$ und $R^{14}$ Wasserstoff und der andere nieder-Alkyl oder Trifluormethyl bedeutet, sowie physiologisch verträgliche Salze von Carbonsäuren der Formel I.

**3.** Verbindungen gemäss Anspruch 1 oder 2, in denen $R^1$ Hydroxy oder nieder-Alkoxy ist.

**4.** Verbindungen gemäss den Ansprüchen 1-3, in denen $R^2$ Wasserstoff ist.

**5.** Verbindungen gemäss den Ansprüchen 1-4, in denen $R^3$, $R^4$, $R^5$, $R^6$, $R^7$, $R^8$, $R^9$, $R^{10}$, $R^{11}$ und $R^{12}$ Wasserstoff oder nieder-Alkyl sind.

**6.** Verbindungen gemäss den Ansprüchen 1-4, in denen mindestens einer der Rest $R^3$-$R^{12}$ nieder-Alkyl ist.

**7.** Verbindungen gemäss den Ansprüchen 1-4, in denen $R^{11}$ und $R^{12}$ gemeinsam eine Oxogruppe darstellen.

**8.** Verbindungen gemäss den Ansprüchen 1-4, in denen $R^{11}$ Wasserstoff und $R^{12}$ Hydroxy bedeuten.

**9.** Verbindungen gemäss den Ansprüchen 1-4, in denen $R^3$ und $R^5$ gemeinsam eine Methylengruppe darstellen.

**10.** Aethyl p-[(E)-2-(6,7,8,9-tetrahydro-7,7-dimethyl-5H-benzocyclohepten-2-yl)propenyl]benzoat.

**11.** Die Verbindungen

Aethyl p-[(E)-2-(6,7,8,9-tetrahydro-9,9-dimethyl-5H-benzocyclohepten-2-yl)propenyl]benzoat;

Aethyl p-[(E)-2-(6,7,8,9-tetrahydro-5,5-dimethyl-5H-benzocyclohepten-2-yl)propenyl]benzoat;

Aethyl p-[(E)-2-(6,7,8,9-tetrahydro-5,9,9-trimethyl-5H-benzocyclohepten-2-yl)propenyl]benzoat;
Aethyl p-[(E)-2-(6,7,8,9-tetrahydro-5,5,9-trimethyl-5H-benzocyclohepten-2-yl)propenyl]benzoat;
Aethyl p-[(E)-2-(6,7,8,9-tetrahydro-5,5,9,9-tetramethyl-5H-benzocyclohepten-2-yl)propenyl]benzoat;
Aethyl p-[(E)-2-(6,7,8,9-tetrahydro-7,7,9-trimethyl-5H-benzocyclohepten-2-yl)propenyl]benzoat;
Methyl p-[(E)-2-(6,7,8,9-tetrahydro-7,7,9,9-tetramethyl-5H-benzocyclohepten-2-yl)propenyl]benzoat;
Aethyl p-[(E)-2-(7-äthyl-6,7,8,9-tetrahydro-7-methyl-5H-benzocyclohepten-2-yl)propenyl]benzoat;
p-[(E)-2-(6,7,8,9-Tetrahydro-5H-benzocyclohepten-2-yl)propenyl]benzoesäure;
p-[(E)-2-(6,7,8,9-Tetrahydro-7,7-dimethyl-5H-benzocyclohepten-2-yl)propenyl]benzoesäure;
p-[(E)-2-(6,7,8,9-Tetrahydro-5,5,9,9-tetramethyl-5H-benzocyclohepten-2-yl)propenyl]benzoesäue;
Aethyl p-[(E)-2-(6,7,8,9-tetrahydro-7-methyl-5H-benzocyclohepten-2-yl)propenyl]benzoat;
p-[(E)-2-(6,7,8,9-Tetrahydro-6,6,8,8-tetramethyl-5H-benzocyclohepten-2-yl)propenyl]benzoesäure;
Methyl p-[(E)-(6,7,8,9-tetrahydro-3,7,7-trimethyl-5H-benzocyclohepten-2-yl)propenyl]benzoat;
Aethyl p-[(E)-2-(6,7,8,9-tetrahydro-9-methyl-5H-benzocyclohepten-2-yl)propenyl]benzoat;
p-[(E)-2-(6,7,8,9-Tetrahydro-7,7,9,9-tetramethyl-5H-benzocyclohepten-2-yl)propenyl]benzoesäure;
p-[(Z)-(6,7,8,9-Tetrahydro-7,7-dimethyl-3-octyl-5H-benzocyclohepten-2-yl)propenyl]benzoesäure;
p-[(Z)-2-(3-Brom-6,7,8,9-tetrahydro-7,7-dimethyl-5H-benzocyclohepten-2-yl)propenyl]benzoesäure;
p-[(Z)-2-(6,7,8,9-Tetrahydro-3,7,7-trimethyl-5H-benzocyclohepten-2-yl)propenyl]benzoesäure und
Aethyl p-[(E)-2-(6,7,8,9-tetrahydro-5H-benzocyclohepten-2-yl)propenyl]benzoat.

**12.** Die Verbindungen
Aethyl p-[(E)-2-[5,6,8,9-tetrahydrospiro[7H-benzocyclohepten-7,1′-cyclopropan]-2-yl]propenyl]-benzoat und
Aethyl p-[(E)-2-[5,6,8,9-tetrahydrospiro[7H-benzocyclohepten-7,1′-cyclopentan]-2-yl]propenyl]-benzoat.

**13.** Die Verbindungen
Aethyl p-[(E)-2-(6,7,8,9-tetrahydro-7-oxo-5H-benzocyclohepten-2-yl)propenyl]benzoat und
Aethyl p-[(E)-2-(6,7,8,9-tetrahydro-6,6,8,8-tetramethyl-7-oxo-5H-benzocyclohepten-2-yl)propenyl]-benzoat.

**14.** Die Verbindungen
p-[(E)-2-(6,7,8,9-Tetrahydro-10-acetoxy-5,9-dimethyl-5,9-methano-5H-benzocyclohepten-2-yl)-propenyl]benzoesäure-äthylester;
p-[(E)-2-(6,7,8,9-Tetrahydro-10-hydroxy-5,9-dimethyl-5,9-methano-5H-benzocyclohepten-2-yl)-propenyl]benzoesäure;
p-[(E)-2-(6,7,8,9-Tetrahydro-5,9-dimethyl-5,9-methano-5H-benzocyclohepten-2-yl)propenyl]-benzoesäure-äthylester und
p-[(E)-2-(6,7,8,9-Tetrahydro-5,9-dimethyl-5,9-methano-5H-benzocyclohepten-2-yl)propenyl]-benzoesäure.

**15.** Verbindungen gemäss den Ansprüchen 1-14 zur Verwendung als Heilmittel.

**16.** Verfahren zur Herstellung von Verbindungen der allgemeinen Formel I, dadurch gekennzeichnet, dass man eine Verbindungen der allgemeinen Formel

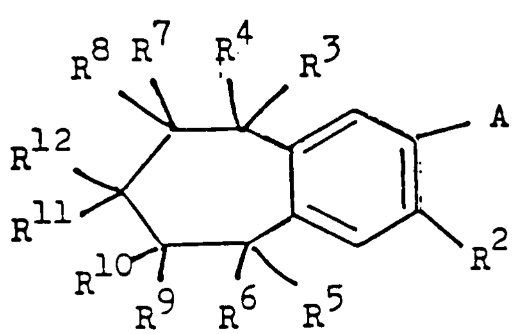

II

mit einer Verbindung der allgemeinen Formel

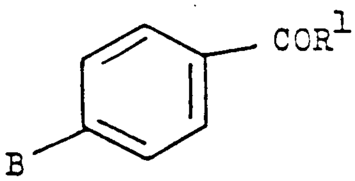

III

umsetzt,
wobei A einen Rest $-CH(R^{13})P^{+}(Q)_3Y^{-}$ und B einen Rest $-CO(R^{14})-$; oder A einen Rest $-CO(R^{13})-$ und B einen Rest $-CH_2-PO(OAlk)_2$; Q Aryl, $Y^{-}$ das Anion einer organischen oder anorganischen Säure, Alk eine niedere Alkylgruppe darstellt und $R^1$-$R^{14}$ die oben angegebene Bedeutung haben und wobei eine als $R^{11}$ oder $R^{11}$ und $R^{12}$ anwesende Hydroxy- bzw. Oxogruppe in geschützter Form vorliegt, und eine gegebenenfalls anwesende Hydroxy- oder Oxoschutzgruppe abspaltet und gewünschtenfalls eine Estergruppe $-COR^1$ im Reaktionsprodukt verseift oder in eine Carboxaminogruppe oder eine N-mono- oder dialkylierte Carboxaminogruppe überführt.

**17.** Pharmazeutische Präparate, enthaltend eine Verbindung gemäss Anspruch 1 und übliche Trägerstoffe.

**18.** Verbindungen gemäss den Ansprüchen 1-14 zur Verwendung als Wirkstoffe in pharmazeutischen Präparaten mit anti-seborrhoischer, anti-keratinisierender, anti-neoplastischer, anti-allergischer und anti-inflammatorischer Wirksamkeit.

**Patentansprüche für folgende Vertragsstaaten : GR, ES**

**1.** Verfahren zur Herstellung von Verbindungen der allgemeinen Formel

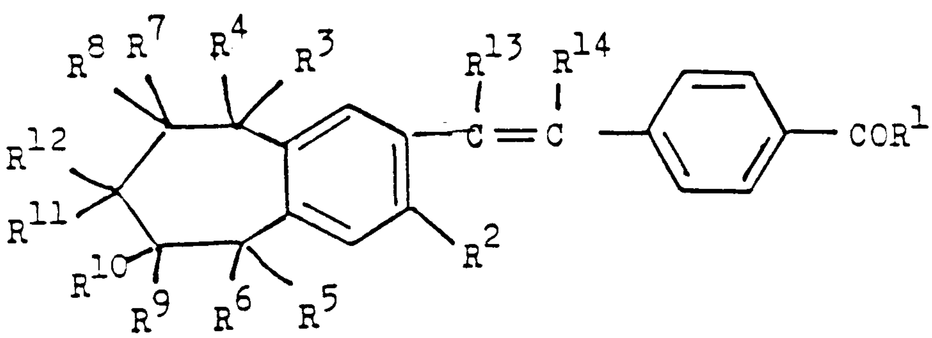

I

worin

| | |
|---|---|
| $R^1$ | Hydroxy, nieder-Alkoxy, Amino, mono- oder di-nieder-Alkylamino; |
| $R^2$ | Wasserstoff, Alkyl, Alkoxy oder Halogen; |
| $R^3$, $R^4$, $R^5$, $R^6$, $R^{11}$ und $R^{12}$ | unabhängig voneinander Wasserstoff oder nieder-Alkyl; |
| $R^3$ und $R^5$ | gemeinsam eine Methylen- oder Hydroxymethylengruppe; |
| $R^7$, $R^8$, $R^9$ und $R^{10}$ | Wasserstoff oder nieder-Alkyl; oder |
| $R^{11}$ und $R^{12}$ | gemeinsam eine Oxogruppe oder Spiro-cyclo-niederalkyl; oder $R^{11}$ Wasserstoff und $R^{12}$ Hydroxy oder Acetoxy; und einer der Reste $R^{13}$ und $R^{14}$ Wasserstoff und der andere nieder-Alkyl oder Trifluormethyl bedeutet, |

sowie physiologisch verträgliche Salze von Carbonsäuren der Formel I, dadurch gekennzeichnet, dass man eine Verbindung der allgemeinen Formel

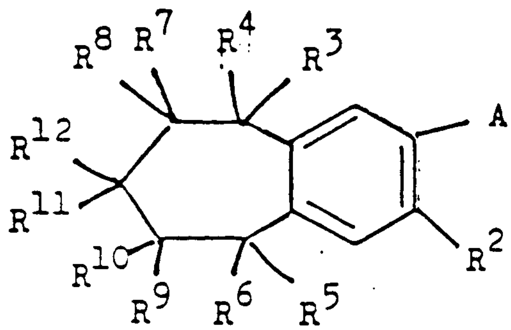

II

mit einer Verbindung der allgemeinen Formel

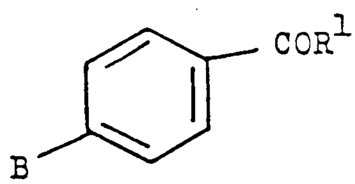

III

umsetzt,
wobei A einen Rest -CH($R^{13}$)$P^+(Q)_3Y^-$ und B einen Rest -CO($R^{14}$)-; oder A einen Rest -CO($R^{13}$)- und B einen Rest $-CH_2-PO(OAlk)_2$; Q Aryl, $Y^-$ das Anion einer organischen oder anorganischen Säure, Alk eine niedere Alkylgruppe darstellt und $R^1$-$R^{14}$ die oben angegebene Bedeutung haben und wobei eine als $R^{11}$ oder $R^{11}$ und $R^{12}$ anwesende Hydroxy- bzw. Oxogruppe in geschützter Form vorliegt, und eine gegebenenfalls anwesende Hydroxy- oder Oxoschutzgruppe abspaltet und gewünschtenfalls eine Estergruppe -COR$^1$ im Reaktionsprodukt verseift oder in eine Carboxaminogruppe oder eine N-mono- oder dialkylierte Carboxaminogruppe überführt; oder eine Carbonsäure der Formel I in ein physiologisch verträgliches Salz überführt.

**2.** Verfahren gemäss Anspruch 1, zur Herstellung von Verbindungen der Formel I worin $R^1$ Hydroxy, nieder-Alkoxy, Amino, mono- oder di-nieder-Alkylamino; $R^2$ Wasserstoff, Alkyl, Alkoxy oder Halogen; $R^3$, $R^4$, $R^5$, $R^6$, $R^{11}$ und $R^{12}$ unabhängig voneinander Wasserstoff oder nieder-Alkyl; $R^3$ und $R^5$ gemeinsam eine Methylengruppe; $R^7$, $R^8$, $R^9$ und $R^{10}$ Wasserstoff oder nieder-Alkyl; $R^{11}$ und $R^{12}$ gemeinsam eine Oxogruppe oder $R^{11}$ Wasserstoff und $R^{12}$ Hydroxy; und einer der Reste der $R^{13}$ und $R^{14}$ Wasserstoff und der andere nieder-Alkyl oder Trifluormethyl bedeutet, sowie physiologisch verträgliche Salze von Carbonsäuren der Formel I.

**3.** Verfahren gemäss Anspruch 1 oder 2, zur Herstellung von Verbindungen der Formel I, in der $R^1$ Hydroxy oder nieder-Alkoxy ist.

**4.** Verfahren gemäss den Ansprüchen 1-3, zur Herstellung von Verbindungen der Formel I, in der $R^2$ Wasserstoff ist.

**5.** Verfahren gemäss den Ansprüchen 1-4, zur Herstellung von Verbindungen der Formel I, in der $R^3$, $R^4$, $R^5$, $R^6$, $R^7$, $R^8$, $R^9$, $R^{10}$, $R^{11}$ und $R^{12}$ Wasserstoff oder nieder-Alkyl sind.

**6.** Verfahren gemäss den Ansprüchen 1-3, zur Herstellung von Verbindungen der Formel I, in der mindestens einer der Reste $R^3$-$R^{12}$ nieder-Alkyl ist.

**7.** Verfahren gemäss den Ansprüchen 1-3, zur Herstellung von Verbindungen der Formel I, in der $R^{11}$ und $R^{12}$ gemeinsam eine Oxogruppe darstellen.

**8.** Verfahren gemäss den Ansprüchen 1-3, zur Herstellung von Verbindungen der Formel I, in der $R^{11}$ Wasserstoff und $R^{12}$ Hydroxy bedeuten.

**9.** Verfahren gemäss den Ansprüchen 1-3, zur Herstellung von Verbindungen der Formel I, in der $R^3$ und $R^5$ gemeinsam eine Methylengruppe darstellen.

**10.** Verfahren gemäss Anspruch 1 zur Herstellung des Aethyl p-[(E)-2-(6,7,8,9-tetrahydro-7,7-dimethyl-5H-benzocyclohepten-2-yl)propenyl]benzoats.

**11.** Verfahren gemäss Anspruch 1 zur Herstellung der Verbindungen
Aethyl p-[(E)-2-(6,7,8,9-tetrahydro-9,9-dimethyl-5H-benzocyclohepten-2-yl)propenyl]benzoat;
Aethyl p-[(E)-2-(6,7,8,9-tetrahydro-5,5-dimethyl-5H-benzocyclohepten-2-yl)propenyl]benzoat;
Aethyl p-[(E)-2-(6,7,8,9-tetrahydro-5,9,9-trimethyl-5H-benzocyclohepten-2-yl)propenyl]benzoat;
Aethyl p-[(E)-2-(6,7,8,9-tetrahydro-5,5,9-trimethyl-5H-benzocyclohepten-2-yl)propenyl]benzoat;
Aethyl p-[(E)-2-(6,7,8,9-tetrahydro-5,5,9,9-tetramethyl-5H-benzocyclohepten-2-yl)propenyl]benzoat;
Aethyl p-[(E)-2-(6,7,8,9-tetrahydro-7,7,9-trimethyl-5H-benzocyclohepten-2-yl)propenyl]benzoat;
Methyl p-[(E)-2-(6,7,8,9-tetrahydro-7,7,9,9-tetramethyl-5H-benzocyclohepten-2-yl)propenyl]benzoat;
Aethyl p-[(E)-2-(7-äthyl-6,7,8,9-tetrahydro-7-methyl-5H-benzocyclohepten-2-yl)propenyl]benzoat;
p-[(E)-2-(6,7,8,9-Tetrahydro-5H-benzocyclohepten-2-yl)propenyl]benzoesäure;
p-[(E)-2-(6,7,8,9-Tetrahydro-7,7-dimethyl-5H-benzocyclohepten-2-yl)-propenyl]benzoesäure;
p-[(E)-2-(6,7,8,9-Tetrahydro-5,5,9,9-tetramethyl-5H-benzocyclohepten-2-yl)propenyl]benzoesäue;
Aethyl p-[(E)-2-(6,7,8,9-tetrahydro-7-methyl-5H-benzocyclohepten-2-yl)propenyl]benzoat;
p-[(E)-2-(6,7,8,9-Tetrahydro-6,6,8,8-tetramethyl-5H-benzocyclohepten-2-yl)propenyl]benzoesäure;
Methyl p-[(E)-(6,7,8,9-tetrahydro-3,7,7-trimethyl-5H-benzocyclohepten-2-yl)propenyl]benzoat;
Aethyl p-[(E)-2-(6,7,8,9-tetrahydro-9-methyl-5H-benzocyclohepten-2-yl)propenyl]benzoat;
p-[(E)-2-(6,7,8,9-Tetrahydro-7,7,9,9-tetramethyl-5H-benzocyclohepten-2-yl)propenyl]benzoesäure;
p-[(Z)-(6,7,8,9-Tetrahydro-7,7-dimethyl-3-octyl-5H-benzocyclohepten-2-yl)propenyl]benzoesäure;
p-[(Z)-2-(3-Brom-6,7,8,9-tetrahydro-7,7-dimethyl-5H-benzocyclohepten-2-yl)propenyl]benzoesäure;
p-[(Z)-2-(6,7,8,9-Tetrahydro-3,7,7-trimethyl-5H-benzocyclohepten-2-yl)propenyl]benzoesäure und
Aethyl p-[(E)-2-(6,7,8,9-tetrahydro-5H-benzocyclohepten-2-yl)propenyl]benzoat.

**12.** Verfahren gemäss Anspruch 1 zur Herstellung der Verbindungen
Aethyl p-[(E)-2-[5,6,8,9-tetrahydrospiro[7H-benzocyclohepten-7,1′-cyclopropan]-2-yl]propenyl]-benzoat und
Aethyl p-[(E)-2-[5,6,8,9-tetrahydrospiro[7H-benzocyclohepten-7,1′-cyclopentan]-2-yl]propenyl]-benzoat.

**13.** Verfahren gemäss Anspruch 1 zur Herstellung der Verbindungen
Aethyl p-[(E)-2-(6,7,8,9-tetrahydro-7-oxo-5H-benzocyclohepten-2-yl)propenyl]benzoat und
Aethyl p-[(E)-2-(6,7,8,9-tetrahydro-6,6,8,8-tetramethyl-7-oxo-5H-benzocyclohepten-2-yl)propenyl]-benzoat.

**14.** Verfahren gemäss Anspruch 1 zur Herstellung der Verbindungen
p-[(E)-2-(6,7,8,9-Tetrahydro-10-acetoxy-5,9-dimethyl-5,9-methano-5H-benzocyclohepten-2-yl)-propenyl]benzoesäure-äthylester;
p-[E)-2-(6,7,8,9-Tetrahydro-10-hydroxy-5,9-dimethyl-5,9-methano-5H-benzocyclohepten-2-yl)-propenyl]benzoesäure;
p-[(E)-2-(6,7,8,9-Tetrahydro-5,9-dimethyl-5,9-methano-5H-benzocyclohepten-2-yl)propenyl]-benzoesäure-äthylester und
p-[(E)-2-(6,7,8,9-Tetrahydro-5,9-dimethyl-5,9-methano-5H-benzocyclohepten-2-yl)propenyl]-benzoesäure.

**15.** Verfahren zur Herstellung pharmazeutischer Präparate, enthaltend eine Verbindung der Formel I oder ein physiologisch verträgliches Salz einer Carbonsäure der Formel I, dadurch gekennzeichnet, dass man eine solche Verbindung mit üblichen Hilfs- und Trägerstoffen in eine pharmazeutische Anwendungsform bringt.

**16.** Verbindungen der Formel I oder physiologisch verträgliche Salze von Carbonsäuren der Formel I zur Verwendung als Wirkstoffe in pharmazeutischen Präparaten mit anti-seborrhoischer, anti-keratinisierender, anti-neoplastischer, anti-allergischer und anti-inflammatorischer Wirksamkeit.

## Claims

**Claims for the following Contracting States : AT, BE, CH, DE, FR, GB, IT, LI, LU, NL, SE**

**1.** Compounds of the general formula

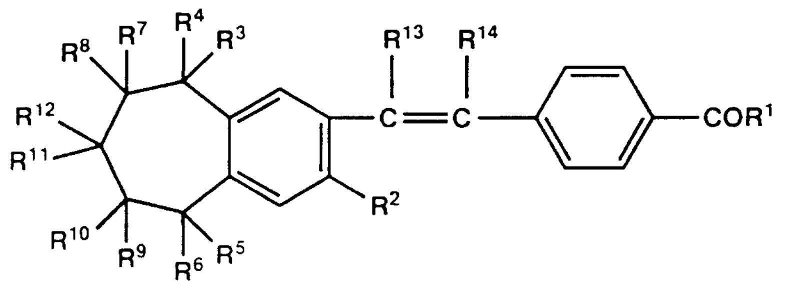

I

wherein

| | |
|---|---|
| $R^1$ | signifies hydroxy, lower-alkoxy, amino mono- or dilower-alkylamino; |
| $R^2$ | signifies hydrogen alkyl, alkoxy or halogen; |
| $R^3$, $R^4$, $R^5$, $R^6$, $R^{11}$ and $R^{12}$ | each independently signify hydrogen or lower-alkyl; |
| $R^3$ and $R^5$ | together signify a methylene or hydroxymethylene group; |
| $R^7$, $R^8$ $R^9$ and $R^{10}$ | signify hydrogen or lower-alkyl; or |
| $R^{11}$ and $R^{12}$ | together signify an oxo group or spiro-cyclo-lower alkyl; or $R^{11}$ signifies hydrogen and $R^{12}$ signifies hydroxy or acetoxy; and one of the residues $R^{13}$ and $R^{14}$ signifies hydrogen and the other signifies lower-alkyl or trifluoromethyl, |

as well as physiologically compatible salts of carboxylic acids of formula I.

**2.** Compounds in accordance with claim 1, in which $R^1$ signifies hydroxy, lower-alkoxy, amino, mono- or di-lower-alkylamino; $R^2$ signifies hydrogen, alkyl, alkoxy or halogen; $R^3$, $R^4$, $R^5$, $R^6$, $R^{11}$ and $R^{12}$ each independently signify hydrogen or lower-alkyl; $R^3$ and $R^5$ together signify a methylene group; $R^7$, $R^8$, $R^9$ and $R^{10}$ signify hydrogen or lower-alkyl; $R^{11}$ and $R^{12}$ together signify an oxo group or $R^{11}$ signifies hydrogen and $R^{12}$ signifies hydroxy; and one of the residues $R^{13}$ and $R^{14}$ signifies hydrogen and the other signifies lower-alkyl or trifluoromethyl, as well as physiologically compatible salts of carboxylic acids of formula I.

**3.** Compounds in accordance with claim 1 or 2, in which $R^1$ is hydroxy or lower-alkoxy.

**4.** Compounds in accordance with claims 1-3, in which $R^2$ is hydrogen.

**5.** Compounds in accordance with claims 1-4, in which $R^3$, $R^4$, $R^5$, $R^6$, $R^7$, $R^8$, $R^9$, $R^{10}$, $R^{11}$ and $R^{12}$ are hydrogen or lower-alkyl.

**6.** Compounds in accordance with claims 1-4, in which at least one of the residues $R^3$-$R^{12}$ is lower-alkyl.

**7.** Compounds in accordance with claims 1-4, in which $R^{11}$ and $R^{12}$ together represent an oxo group.

**8.** Compounds in accordance with claims 1-4, in which $R^{11}$ signifies hydrogen and $R^{12}$ signifies hydroxy.

**9.** Compounds in accordance with claims 1-4, in which $R^3$ and $R^5$ together represent a methylene group.

**10.** Ethyl p-[(E)-2-(6,7,8,9-tetrahydro-7,7-dimethyl-5H-benzocyclohepten-2-yl)propenyl]benzoate.

**11.** The compounds
ethyl p-[(E)-2-(6,7,8,9-tetrahydro-9,9-dimethyl-5H-benzocyclohepten-2-yl)propenyl]benzoate;
ethyl p-[(E)-2-(6,7,8,9-tetrahydro-5,5-dimethyl-5H-benzocyclohepten-2-yl)propenyl]benzoate;
ethyl p-[(E)-2-(6,7,8,9-tetrahydro-5,9,9-trimethyl-5H-benzocyclohepten-2-yl)propenyl]benzoate;
ethyl p-[(E)-2-(6,7,8,9-tetrahydro-5,5,9-trimethyl-5H-benzocyclohepten-2-yl)propenyl]benzoate;

ethyl p-[(E)-2-(6,7,8,9-tetrahydro-5,5,9,9-tetramethyl-5H-benzocyclohepten-2-yl)propenyl]benzoate;
ethyl p-[(E)-2-(6,7,8,9-tetrahydro-7,7,9-trimethyl-5H benzocyclohepten-2-yl)propenyl]benzoate;
methyl p-[(E)-2-(6,7,8,9-tetrahydro-7,7,9,9-tetramethyl-5H-benzocyclohepten-2-yl)propenyl]benzoate;
ethyl p-[(E)-2-(7-ethyl-6,7,8,9-tetrahydro-7-methyl-5H-benzocyclohepten-2-yl)propenyl]benzoate;
p-[(E)-2-(6,7,8,9-tetrahydro-5H-benzocyclohepten-2-yl)propenyl]benzoic acid;
P-[(E)-2-(6,7,8,9-tetrahydro-7,7-dimethyl-5H-benzocyclohepten-2-yl)propenyl]benzoic acid;
p-[(E)-2-(6,7,8,9-tetrahydro-5,5,9,9-tetramethyl-5H-benzocycohepten-2-yl)propenyl]benzoic acid;
ethyl p-[(E)-2-(6,7,8,9-tetrahydro-7-methyl-5H-benzocyclohepten-2-yl)propenyl]benzoate;
p-[(E)-2-(6,7,8,9-tetrahydro-6,6,8,8-tetramethyl-5H-benzocyclohepten-2-yl)propenyl]benzoic acid;
methyl p-[(E)-(6,7,8,9-tetrahydro-3,7,7-trimethyl-5H-benzocyclohepten-2-yl)propenyl]benzoate;
ethyl p-[(E)-2-(6,7,8,9-tetrahydro-9-methyl-5H-benzocyclohepten-2-yl)propenyl]benzoate;
p-[(E)-2-(6,7,8,9-tetrahydro-7,7,9,9-tetramethyl-5H-benzocyclohepten-2-yl)propenyl]benzoic acid;
p-[(Z)-(6,7,8,9-tetrahydro-7,7-dimethyl-3-octyl-5H-benzocyclohepten-2-yl)propenyl]benzoic acid;
p-[(Z)-2-(3-bromo-6,7-8,9-tetrahydro-7,7-dimethyl-5H-benzocyclohepten-2-yl)propenyl]benzoic acid;
p-[(Z)-2-(6,7,8,9-tetrahydro-3,7,7-trimethyl-5H-benzocyclohepten-2-yl)propenyl]benzoic acid and
ethyl p-[(E)-2-(6,7,8,9-tetrahydro-5H-benzocyclohepten-2-yl)propenyl]benzoate.

**12.** The compounds
ethyl p-[(E)-2-[5,6,8,9-tetrahydrospiro[7H-benzocyclohepten-7,1'-cyclopropane]-2-yl]propenyl]-benzoate and
ethyl p-[(E)-2-[5,6,8,9-tetrahydrospiro[7H-benzocyclohepten-7,1'-cyclopentane]-2-yl]propenyl]-benzoate.

**13.** The compounds
ethyl p-[(E)-2-(6,7,8,9-tetrahydro-7-oxo-5H-benzocyclohepten-2-yl)propenyl]benzoate and
ethyl p-[(E)-2-(6,7,8,9-tetrahydro-6,6,8,8-tetramethyl-7-oxo-5H-benzocyclohepten-2-yl)propenyl]-benzoate.

**14.** The compounds
ethyl p-[(E)-2-(6,7,8,9-tetrahydro-10-acetoxy-5,9-dimethyl-5,9-methano-5H-benzocyclohepten-2-yl)-propenyl]-benzoate;
p-[(E)-2-(6,7,8,9-tetrahydro-10-hydroxy-5,9-dimethyl-5,9-methano-5H-benzocyclohepten-2-yl)-propenyl]benzoic acid;
ethyl p-[(E)-2-(6,7,8,9-tetrahydro-5,9-dimethyl-5.9-methano-5H-benzocyclohepten-2-yl)propenyl]-benzoate and
p-[(E)-2-(6,7,8,9-tetrahydro-5,9-dimethyl-5,9-methano-5H-benzocyclohepten-2-yl)propenyl]benzoic acid.

**15.** Compounds in accordance with claims 1-14 for use as medicaments.

**16.** A process for the manufacture of compounds of general formula I, characterized by reacting a compound of the general formula

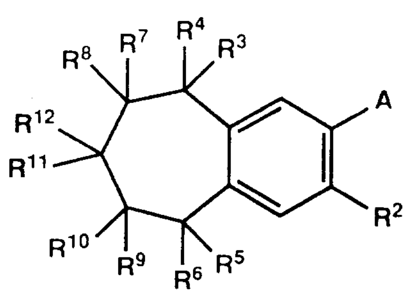

II

with a compound of the general formula

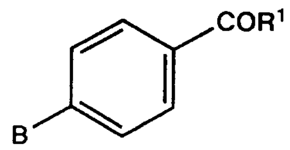

III

wherein A represents a residue -$CH(R^{13})P^+(Q)_3Y^-$ and B represents a residue -$CO(R^{14})$-; or A represents a residue -$CO(R^{13})$- and B represents a residue -$CH_2$- -$PO(OAlk)_2$; Q represents aryl, $Y^-$ represents the anion of an organic or inorganic acid, Alk represents a lower alkyl group and $R^1$-$R^{14}$ have the significance given above and wherein a hydroxy or oxo group present as $R^{11}$ or $R^{11}$ and $R^{12}$ is present in protected form, cleaving off from the condensation product a hydroxy or oxo protecting group which may be present and, if desired, saponifying an ester group -$COR^1$ of the thus-obtained compound of formula I or converting it into a carboxamino group or a N-mono- or dialkylated carboxamino group.

**17.** Pharmaceutical preparations, containing a compound in accordance with claim 1 and usual carrier substances.

**18.** Compounds in accordance with claims 1-14 for use as active substances in pharmaceutical preparations having anti-seborrhoic, anti-keratinizing, anti-neoplastic, anti-allergic and anti-inflammatory activity.

**Claims for the following Contracting States : GR, ES**

**1.** A process for the manufacture of compounds of the general formula

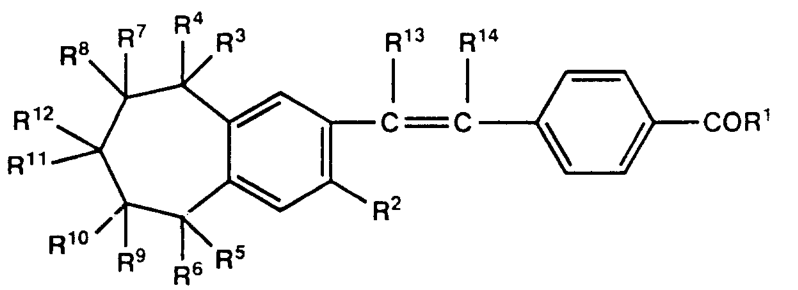

I

wherein

| | |
|---|---|
| $R^1$ | signifies hydroxy, lower-alkoxy, amino, mono- or dilower-alkylamino, |
| $R^2$ | signifies hydrogen, alkyl, alkoxy or halogen; |
| $R^3$, $R^4$, $R^5$, $R^6$, $R^{11}$ and $R^{12}$ | each independently signify hydrogen or lower-alkyl; |
| $R^3$ and $R^5$ | together signify a methylene or hydroxy-methylene group; |
| $R^7$, $R^8$, $R^9$ and $R^{10}$ | signify hydrogen or lower-alkyl: or |
| $R^{11}$ and $R^{12}$ | together signify an oxo group or spiro-cyclo-lower alkyl; or $R^{11}$ signifies hydrogen and $R^{12}$ signifies hydroxy or acetoxy; and one of the residues $R^{13}$ and $R^{14}$ signifies hydrogen and the other signifies lower-alkyl or trifluoromethyl, |

as well as physiologically compatible salts of carboxylic acids of formula I, characterized by reacting a compound of the general formula

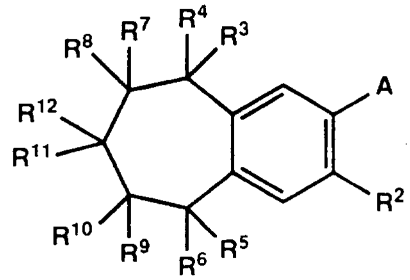

II

with a compound of the general formula

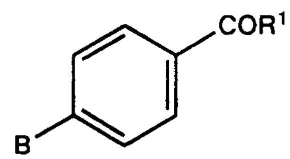

III

wherein A represents a residue $-CH(R^{13})P^{+}(Q)_3Y^{-}$ and B represents a residue $-CO(R^{14})$-; or A represents a residue $-CO(R^{13})$- and B represents a residue $-CH_2$- $-PO(OAlk)_2$; Q represents aryl, $Y^{-}$ represents the anion of an organic or inorganic acid, Alk represents a lower alkyl group and $R^1$-$R^{14}$ have the significance given above and wherein a hydroxy or oxo group present as $R^{11}$ or $R^{11}$ and $R^{12}$ is present in protected form, cleaving off from the condensation product a hydroxy or oxo protecting group which may be present and, if desired, saponifying an ester group $-COR^1$ of the thus-obtained compound of formula I or converting it into a carboxamino group or a N-mono- or dialkylated carboxamino group; or converting a carboxylic acid of formula I into a physiologically compatible salt.

**2.** A process in accordance with claim 1 for the manufacture of compounds of formula I in which $R^1$ signifies hydroxy, lower-alkoxy, amino, mono- or di-lower-alkylamino; $R^2$ signifies hydrogen, alkyl, alkoxy or halogen: $R^3$, $R^4$, $R^5$, $R^6$, $R^{11}$ and $R^{12}$ each independently signify hydrogen or lower-alkyl; $R^3$ and $R^5$ together signify a methylene group; $R^7$, $R^8$, $R^9$ and $R^{10}$ signify hydrogen or lower-alkyl; $R^{11}$ and $R^{12}$ together signify an oxo group or $R^{11}$ signifies hydrogen and $R^{12}$ signifies hydroxy; and one of the residues $R^{13}$ and $R^{14}$ signifies hydrogen and the other signifies lower-alkyl or trifluoromethyl, as well as physiologically compatible salts of carboxylic acids of formula I.

**3.** A process in accordance with claim 1 or 2 for the manufacture of compounds of formula I in which $R^1$ is hydroxy or lower-alkoxy.

**4.** A process in accordance with claims 1-3 for the manufacture of compounds of formula I in which $R^2$ is hydrogen.

**5.** A process in accordance with claims 1-4 for the manufacture of compounds of formula I in which $R^3$, $R^4$, $R^5$, $R^6$, $R^7$, $R^8$, $R^9$, $R^{10}$, $R^{11}$ and $R^{12}$ are hydrogen or lower-alkyl.

**6.** A process in accordance with claims 1-3 for the manufacture of compounds of formula I in which at least one of the residues $R^3$-$R^{12}$ is lower-alkyl.

**7.** A process in accordance with claims 1-3 for the manufacture of compounds of formula I in which $R^{11}$ and $R^{12}$ together represent an oxo group.

**8.** A process in accordance with claims 1-3 for the manufacture of compounds of formula I in which $R^{11}$ signifies hydrogen and $R^{12}$ signifies hydroxy.

**9.** A process in accordance with claims 1-3 for the manufacture of compounds of formula I in which $R^3$ and $R^5$ together represent a methylene group.

**10.** A process according to claim 1 for the manufacture of ethyl p-[(E)-2-(6,7,8,9-tetrahydro-7,7-dimethyl-5H-benzocyclohepten-2-yl)propenyl]benzoate.

**11.** A process according to claim 1 for the manufacture of the compounds
ethyl p-[(E)-2-(6,7,8,9-tetrahydro-9,9-dimethyl-5H-benzocyclohepten-2-yl)propenyl]benzoate;
ethyl p-[(E)-2-(6,7,8,9-tetrahydro-5,5-dimethyl-5H-benzocyclohepten-2-yl)propenyl]benzoate;
ethyl p-[(E)-2-(6,7,8,9-tetrahydro-5,9,9-trimethyl-5H-benzocyclohepten-2-yl)propenyl]benzoate;
ethyl p-[(E)-2-(6,7,8,9-tetrahydro-5,5,9-trimethyl-5H-benzocyclohepten-2-yl)propenyl]benzoate;
ethyl p-[(E)-2-(6,7,8,9-tetrahydro-5,5,9,9-tetramethyl-5H-benzocyclohepten-2-yl)propenyl]benzoate;
ethyl p-[(E)-2-(6,7,8,9-tetrahydro-7,7,9-trimethyl-5H-benzocyclohepten-2-yl)propenyl]benzoate;
methyl p-[(E)-2-(6,7,8,9-tetrahydro-7,7,9,9-tetramethyl-5H-benzocyclohepten-2-yl)propenyl]benzoate;
ethyl p-[(E)-2-(7-ethyl-6,7,8,9-tetrahydro-7-methyl-5H-benzocyclohepten-2-yl)propenyl]benzoate;
p-[(E)-2-(6,7,8,9-tetrahydro-5H-benzocyclohepten-2-yl)propenyl]benzoic acid;
p-[(E)-2-(6,7,8,9-tetrahydro-7,7-dimethyl-5H-benzocyclohepten-2-yl)propenyl]benzoic acid;
p-[(E)-2-(6,7,8,9-tetrahydro-5,5,9,9-tetramethyl-5H-benzocyclohepten-2-yl)propenyl]benzoic acid;
ethyl p-[(E)-2-(6,7,8,9-tetrahydro-7-methyl-5H-benzocyclohepten-2-yl)propenyl]benzoate;
p-[(E)-2-(6,7,8,9-tetrahydro-6,6,8,8-tetramethyl-5H-benzocyclohepten-2-yl)propenyl]benzoic acid;
methyl p-[(E)-(6,7,8,9-tetrahydro-3,7,7-trimethyl-5H-benzocyclohepten-2-yl)propenyl]benzoate;
ethyl p-[(E)-2-(6,7,8,9-tetrahydro-9-methyl-5H-benzocyclohepten-2-yl)propenyl]benzoate;
p-[(E)-2-(6,7,8,9-tetrahydro-7,7,9,9-tetramethyl-5H-benzocyclohepten-2-yl)propenyl]benzoic acid;
p-[(Z)-(6,7,8,9-tetrahydro-7,7-dimethyl-3-octyl-5H-benzocyclohepten-2-yl)propenyl]benzoic acid;
p-[(Z)-2-(3-bromo-6,7-8,9-tetrahydro-7,7-dimethyl-5H-benzocyclohepten-2-yl)propenyl]benzoic acid;
p-[(Z)-2-(6,7,8,9-tetrahydro-3,7,7-trimethyl-5H-benzocyclohepten-2-yl)propenyl]benzoic acid and
ethyl p-[(E)-2-(6,7,8,9-tetrahydro-5H-benzocyclohepten-2-yl)propenyl]benzoate.

**12.** A process according to claim 1 for the manufacture of the compounds
ethyl p-[(E)-2-[5,6,8,9-tetrahydrospiro[7H-benzocyclohepten-7,1'-cyclopropane]-2-yl]propenyl]-benzoate and
ethyl p-[(E)-2-[5,6,8,9-tetrahydrospiro[7H-benzocyclohepten-7,1'-cyclopentane]-2-yl]propenyl]-benzoate.

**13.** A process according to claim 1 for the manufacture of the compounds
ethyl p-[(E)-2-(6,7,8,9-tetrahydro-7-oxo-5H-benzocyclohepten-2-yl)propenyl]benzoate and
ethyl p-[(E)-2-(6,7,8,9-tetrahydro-6,6,8,8-tetramethyl-7-oxo-5H-benzocyclohepten-2-yl)propenyl]-benzoate.

**14.** A process according to claim 1 for the manufacture of the compounds
ethyl p-[(E)-2-(6,7,8,9-tetrahydro-10-acetoxy-5,9-dimethyl-5,9-methano-5H-benzocyclohepten-2-yl)-propenyl]benzoate;
p-[(E)-2-(6,7,8,9-tetrahydro-10-hydroxy-5,9-dimethyl-5,9-methano-5H-benzocyclohepten-2-yl)-propenyl]benzoic acid;
ethyl p-[(E)-2-(6,7,8,9-tetrahydro-5,9-dimethyl-5,9-methano-5H-benzocyclohepten-2-yl)propenyl]-benzoate and
p-[(E)-2-(6,7,8,9-tetrahydro-5,9-dimethyl-5,9-methano-5H-benzocyclohepten-2-yl)propenyl]benzoic acid.

**15.** A process for the manufacture of pharmaceutical preparations containing a compound of formula I or a physiologically compatible salt of a carboxylic acid of formula I, characterized by bringing such a compound into a pharmaceutical administration form with usual adjuvants and carriers.

**16.** Compounds of formula I or physiologically compatible salts of carboxylic acids of formula I for use as active substances in pharmaceutical preparations having anti-seborrhoic, anti-kerantinizing, anti-neoplastic, anti-allergic and anti-inflammatory activity.

**Revendications**

**Revendications pour les Etats contractants suivants : AT, BE, CH, DE, FR, GB, IT, LI, LU, NL, SE**

**1.** Composés de formule générale

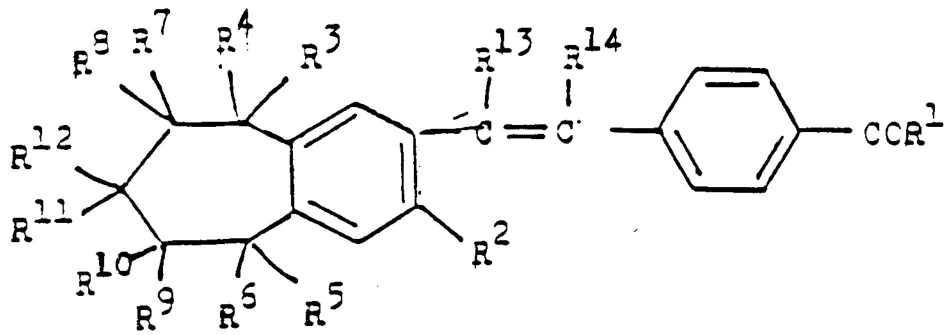

I

où

| | |
|---|---|
| $R^1$ | représente l'hydroxy, un alcoxy inférieur, un amino, un mono- ou dialkyle inférieur amino; |
| $R^2$ | l'hydrogène, un alkye, un alcoxy ou un halogène; |
| $R^3$, $R^4$, $R^5$, $R^6$, $R^{11}$ et $R^{12}$ | représentent indépendamment les uns des autres, l'hydrogène ou un alkyle inférieur; |
| $R^3$ et $R^5$ | représentent ensemble un groupe méthylène ou un groupe hydroxy-méthylène; |
| $R^7$, $R^8$, $R^9$ et $R^{10}$ | représentent l'hydrogène ou un alkyle inférieur; ou |
| $R^{11}$ et $R^{12}$ | représentent ensemble un groupe oxo ou un spiro-cycloalkyle inférieur; ou $R^{11}$ représente l'hydrogène et $R^{12}$ l'hydroxy ou l'acétoxy; et l'un des restes $R^{13}$ et $R^{14}$ représente l'hydrogène et l'autre reste un alkyle inférieur ou le trifluorométhyle, |

ainsi que les sels physiologiquement compatibles des acides carboxyliques de formule I.

**2.** Composés selon la revendication 1 où $R^1$ représente l'hydroxy, un alcoxy inférieur, un amino, un mono- ou dialkyle inférieur amino; $R^2$ l'hydrogène, un alkyle, un alcoxy ou un halogène; $R^3$, $R^4$, $R^5$, $R^6$, $R^{11}$ et $R^{12}$ représentent, indépendamment les uns des autres, l'hydrogène ou un alkyle inférieur; $R^3$ et $R^5$ représentent ensemble un groupe méthylène; $R^7$, $R^8$, $R^9$ et $R^{10}$ l'hydrogène ou un alkyle inférieur; $R^{11}$ et $R^{12}$ représentent ensemble un groupe oxo ou $R^{11}$ représente l'hydrogène et $R^{12}$ l'hydroxy; et l'un des restes $R^{13}$ et $R^{14}$ représente l'hydrogène et l'autre reste un alkyle inférieur ou le trifluorométhyle, ainsi que les sels physiologiquement compatibles des acides carboxyliques de formule I.

**3.** Composés selon la revendication 1 ou 2, dans lesquels $R^1$ est l'hydroxy ou un alcoxy inférieur.

**4.** Composés selon les revendications 1 à 3, dans lesquels $R^2$ est l'hydrogène.

**5.** Composés selon les revendications 1 à 4, dans lesquels $R^3$, $R^4$, $R^5$, $R^6$, $R^7$, $R^8$, $R^9$, $R^{10}$, $R^{11}$ et $R^{12}$ sont l'hydrogène ou un alkyle inférieur.

**6.** Composés selon les revendications 1 à 4, dans lesquels au moins l'un des restes $R^3$ à $R^{12}$ est un alkyle inférieur.

**7.** Composés selon les revendications 1 à 4, dans lesquels $R^{11}$ et $R^{12}$ représentent ensemble un groupe oxo.

**8.** Composés selon les revendications 1 à 4, dans lesquels $R^{11}$ représente l'hydrogène et $R^{12}$ l'hydroxy.

**9.** Composés selon les revendications 1 à 4, dans lesquels $R^3$ et $R^5$ représentent ensemble un groupe méthylène.

**10.** Le benzoate d'éthyle p-[(E)-2-(6,7,8,9-tétrahydro-7,7-diméthyl-5H-benzocycloheptén-2-yl)propényle].

**11.** Les composés

benzoate d'éthyle p-[(E)-2-(6,7,8,9-tétrahydro-9,9-diméthyl-5H-benzocycloheptén-2-yl)propényle];

benzoate d'éthyle p-[(E)-2-(6,7,8,9-tétrahydro-5,5-diméthyl-5H-benzocycloheptén-2-yl)propényle];

benzoate d'éthyle p-[(E)-2-(6,7,8,9-tétrahydro-5,9,9-trimétpyl-5H-benzocycloheptén-2-yl)propényle];

benzoate d'éthylep-[(E)-2-(6,7,8,9-tétrahydro-5,5,9-triméthyl-5H-benzocycloheptén-2-yl)propényle];

benzoate d'éthyle p-[(E)-2-(6,7,8,9-tétrahydro-5,5,9,9-tétraméthyl-5H-benzocycloheptén-2-yl)-propényle];

benzoate d'éthyle p-[(E)-2-(6,7,8,9-tétrahydro-7,7,9-triméthyl-5H-benzocycloheptén-2-yl)propényle];

benzoate de méthyle p-[(E)-2-(6,7,8,9-tétrahydro-7,7,9,9-tétraméthyl-5H-benzocycloheptén-2-yl)-propényle];

benzoate d'éthyle p-[(E)-2-(7-éthyl-6,7,8,9-tétrahydro-7-méthyl-5H-benzocycloheptén-2-yl)-propényle];

acide p-[(E)-2-(6,7,8,9-tétrahydro-5H-benzocycloheptén-2-yl)-propényl]benzoïque;

acide p-[(E)-2-(6,7,8,9-tétrahydro-7,7-diméthyl-5H-benzocycloheptén-2-yl)propényl]benzoïque;

acide p-[(E)-2-(6,7,8,9-tétrahydro-5,5,9,9-tétraméthyl-5H-benzocycloheptén-2-yl)propényl]benzoïque;

benzoate d'éthyle p-[(E)-2-(6,7,8,9-tétrahydro-7-méthyl-5H-benzocycloheptén-2-yl)propényle];

acide p-[(E)-2-(6,7,8,9-tétrahydro-6,6,8,8-tétraméthyl-5H-benzocycloheptén-2-yl)propényl]benzoïque;

benzoate de méthyle p-[(E)-(6,7,8,9-tétrahydro-3,7,7-triméthyl-5H-bensocycloheptén-2-yl)-propényle];

benzoate d'éthyle p-[(E)-2-(6,7,8,9-tétrahydro-9-méthyl-5H-benzocycloheptén-2-yl)propényle];

acide p-[(E)-2-(6,7,8,9-tétrahydro-7,7,9,9-tétraméthyl-5H-benzocyçloheptén-2-yl)propényl]-benzoïque;

acide p-[(Z)-(6,7,8,9-tétrahydro-7,7-diméthyl-3-octyl-5H-benzocycloheptén-2-yl)propényl]benzoïque;

acide p-[(Z)-2-(3-bromo-6,7,8,9-tétrahydro-7,7-diméthyl-5H-benzocycloheptén-2-yl)propényl]-benzoïque;

acide p-[(Z)-2-(6,7,8,9-tétrahydro-3,7,7-triméthyl-5H-benzocyloheptén-2-yl)propényl]benzoïque et

benzoate d'éthyle p-[(E)-2-(6,7,8,9-tétrahydro-5H-benzocycloheptén-2-yl)propényle].

**12.** Les composés

benzoate d'éthyle p-[(E)-2-[5,6,8,9-tétrahydrospiro[7H-benzocycloheptène-7,1'-cyclopropan]-2-yl]-propényle] et

benzoate d'éthyle p-[(E)-2-[5,6,8,9-tétrahydrospiro[7H-benzocyclohepténe-7,1'-cyclopentan]-2-yl]-propényle].

**13.** Les composés

benzoate d'éthyle p-[(E)-2-(6,7,8,9-tétrahydro-7-oxo-5H-benzocycloheptén-2-yl)propényle] et

benzoate d'éthyle p-[(E)-2-(6,7,8,9-tétrahydro-6,6,8,8-tétraméthyl-7-oxo-5H-benzocycloheptén-2-yl)-propényle].

**14.** Les composés

ester éthylique de l'acide p-[(E)-2-(6,7,8,9-tétrahydro-10-acétoxy-5,9-diméthyl-5,9-méthano-5H-benzocycloheptén-2-yl)propényl]benzoïque;

acide p-[(E)-2-(6,7,8,9-tétrahydro-10-hydroxy-5,9-diméthyl-5,9-méthano-5H-benzocycloheptén-2-yl)-propényl]benzoïque;

ester éthylique de l'acide p-[(E)-2-(6,7,8,9-tétrahydro-5,9-diméthyl-5,9-méthano-5H-benzocycloheptén-2-yl)propényl]benzoïque et

acide p-[(E)-2-(6,7,8,9-tétrahydro-5,9-diméthyl-5,9-méthano-5H-benzocycloheptén-2-yl)propényl]-benzoïque.

**15.** Composés selon les revendications 1 à 14 destinés à être utilisés comme médicaments.

**16.** Procédé pour la préparation des composés de formule générale I, caractérisé en ce que l'on fait réagir un composé de formule générale

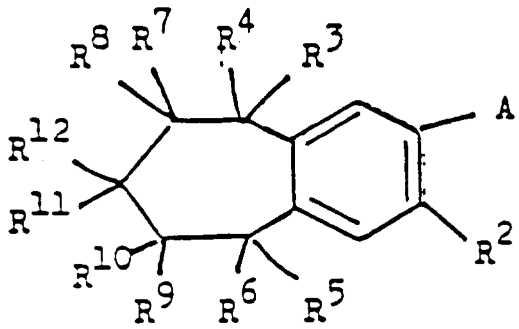

II

avec un composé de formule générale

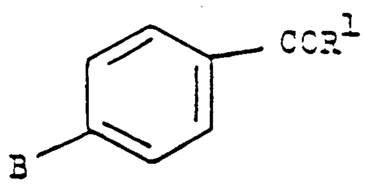

III

où A représente un reste $-CH(R^{13})P^{+}(Q)_3Y^{-}$ et B un reste - $CO(R^{14})$-; ou A représente un reste -CO-($R^{13}$)- et B représente un reste $-CH_2-PO(OAlk)_2$; Q représente un aryle, $Y^{-}$ représente l'anion d'un acide organique ou minéral, Alk un groupe alkyle inférieur et $R^1$ à $R^{14}$ ont les significations indiquées ci-dessus et où un groupe hydroxy ou oxo présent pour $R^{11}$ ou $R^{11}$ et $R^{12}$ se trouve sous forme protégée, en ce que l'on clive le groupe protecteur de l'hydroxy ou de l'oxo éventuellement présent et, si désiré, en ce que l'on saponifie un groupe ester $-COR^1$ dans le produit de réaction ou en ce qu'on le transforme en un groupe carboxamino ou en un groupe carboxamino N-mono- ou dialkylé.

**17.** Préparations pharmaceutiques contenant un composé selon la revendication 1 et des supports usuels.

**18.** Composés selon les revendications 1 à 14 destinés à être utilisés comme substances actives dans les préparations pharmaceutiques présentant une activité anti-séborrhéique, anti-kératinisante, anti-néoplastique, anti-allergique et anti-inflammatoire.

**Revendications pour les Etats contractants suivants : GR, ES**

**1.** Procédé pour la préparation des composés de formule générale

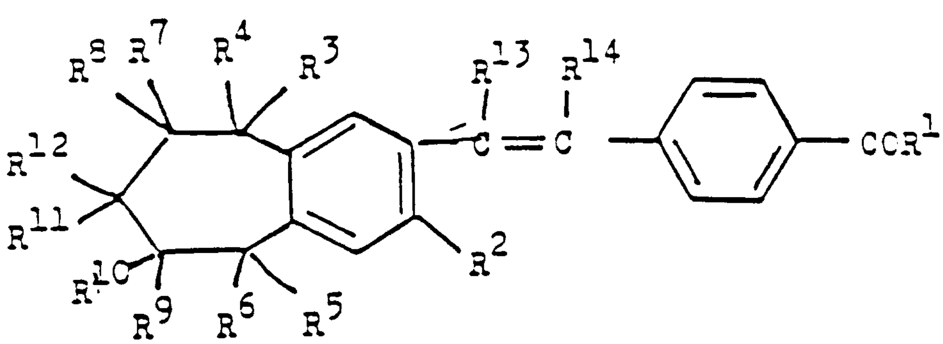

I

où

| | |
|---|---|
| $R^1$ | représente l'hydroxy, un alcoxy inférieur, un amino, un mono- ou dialkyle inférieur amino; |
| $R^2$ | l'hydrogène, un alkyle, un alcoxy ou un halogène; |
| $R^3$, $R^4$, $R^5$, $R^6$, $R^{11}$ et $R^{12}$ | représentent indépendamment les uns des autres, l'hydrogène ou un alkyle inférieur; |

| | |
|---|---|
| $R^3$ et $R^5$ | représentent ensemble un groupe méthylène ou un groupe hydroxyméthylène; |
| $R^7$, $R^8$, $R^9$ et $R^{10}$ | représentent l'hydrogène ou un alkyle inférieur; ou |
| $R^{11}$ et $R^{12}$ | représentent ensemble un groupe oxo ou un spiro-cycloalkyle inférieur; ou $R^{11}$ représente l'hydrogène et $R^{12}$ l'hydroxy ou l'acétoxy; et l'un des restes $R^{13}$ et $R^{14}$ représente l'hydrogène et l'autre reste un alkyle inférieur ou le trifluorométhyle, |

ainsi que les sels physiologiquement compatibles des acides carboxyliques de formule I, caractérisé en ce que l'on fait réagir un composé de formule générale

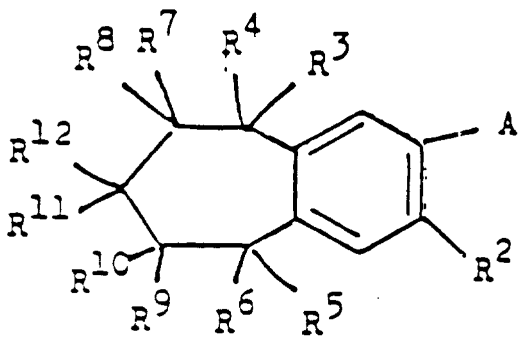

II

avec un composé de formule générale

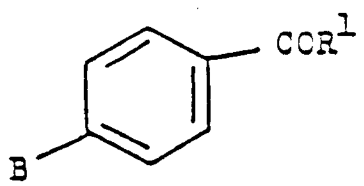

III

où A représente un reste $-CH(R^{13})P^{+}(Q)_3Y^{-}$ et B un reste $-CO(R^{14})-$; ou A représente un reste $-CO-(R^{13})-$ et B représente un reste $-CH_2-PO(OAlk)_2$; Q représente un aryle, $Y^{-}$ représente l'anion d'un acide organique ou minéral, Alk un groupe alkyle inférieur et $R^1$ à $R^{14}$ ont les significations indiquées ci-dessus et où un groupe hydroxy ou oxo présent pour $R^{11}$ ou $R^{11}$ et $R^{12}$ se trouve sous forme protégée, en ce que l'on clive le groupe protecteur de l'hydroxy ou de l'oxu éventuellement présent et, si désiré, en ce que l'on saponifie un groupe ester $-COR^1$ dans le produit de réaction ou en ce qu'on le transforme en un groupe carboxamino ou en un groupe carboxamino N-mono- ou dialkylé, ou en ce que l'on transforme un acide carboxylique de formule I en un sel physiologiquement compatible.

**2.** Procédé selon la revendication 1 pour la préparation des composés de formule I où $R^1$ représente l'hydroxy, un alcoxy inférieur, un amino, un mono- ou dialkyle inférieur amino; $R^2$ l'hydrogène, un alkyle, un alcoxy ou un halogène; $R^3$, $R^4$, $R^5$, $R^6$, $R^{11}$ et $R^{12}$ représentent, indépendamment les uns des autres, l'hydrogène ou un alkyle inférieur; $R^3$ et $R^5$ représentent ensemble un groupe méthylène; $R^7$, $R^8$, $R^9$ et $R^{10}$ l'hydrogène ou un alkyle inférieur; $R^{11}$ et $R^{12}$ représentent ensemble un groupe oxo ou $R^{11}$ représente l'hydrogène et $R^{12}$ l'hydroxy; et l'un des restes $R^{13}$ et $R^{14}$ représente l'hydrogène et l'autre reste un alkyle inférieur ou le trifluorométhyle, ainsi que les sels physiologiquement compatibles des acides carboxyliques de formule I.

**3.** Procédé selon la revendication 1 ou 2, pour la préparation des composés de formule I dans laquelle $R^1$ est l'hydroxy ou un alcoxy inférieur.

**4.** Procédé selon les revendications 1 à 3, pour la préparation des composés de formule I dans laquelle $R^2$ est l'hydrogène.

**5.** Procédé selon les revendications 1 à 4, pour la préparation des composés de formule I, dans laquelle $R^3$, $R^4$, $R^5$, $R^6$, $R^7$, $R^8$, $R^9$, $R^{10}$, $R^{11}$ et $R^{12}$ sont l'hydrogène ou un alkyle inférieur.

6. Procédé selon les revendications 1 à 3, pour la préparation des composés de formule I dans laquelle au moins l'un des restes $R^3$ à $R^{12}$ est un alkyle inférieur.

7. Procédé selon les revendications 1 à 3, pour la préparation des composés de formule I dans laquelle $R^{11}$ et $R^{12}$ représentent ensemble un groupe oxo.

8. Procédé selon les revendications 1 à 3, pour la préparation des composés de formule I dans laquelle $R^{11}$ représente l'hydrogène et $R^{12}$ l'hydroxy.

9. Procédé selon les revendications 1 à 3, pour la préparation des composés de formule I dans laquelle $R^3$ et $R^5$ représentent ensemble un groupe méthylène.

10. Procédé selon la revendication 1, pour la préparation du benzoate d'éthyle p-[(E)-2-(6,7,8,9-tétrahydro-7,7-diméthyl-5H-benzcyclohepten-2-yl)propényle].

11. Procédé selon la revendication 1 pour la préparation des composés
benzoate d'éthyle p-[(E)-2-(6,7,8,9-tétrahydro-9,9-diméthyl-5H-benzocycloheptén-2-yl)propényle];
benzoate d'éthyle p-[(E)-2-(6,7,8,9-tétrahydro-5,5-diméthyl-5H-benzocyclohepten-2-yl)propényle];
benzoate d'éthyle p-[(E)-2-(6,7,8,9-tétrahydro-5,9,9-triméthyl-5H-benzocyclohepten-2-yl)propényl];
benzoate d'éthyle p-[(E)-2-(6,7,8,9-tétrahydro-5,5,9-triméthyl-5H-benzocyclohepten-2-yl)propényle];
benzoate d'éthyle p-[(E)-2-(6,7,8,9-tétrahydro-5,5,9,9-tétraméthyl-5H-benzocyclohepten-2-yl)-propényle];
benzoate d'éthyle p-[(E)-2-(6,7,8,9-tétrahydro-7,7,9-triméthyl-5H-benzocyclohepten-2-yl)propényle];
benzoate de méthyle p-[(E)-2-(6,7,8,9-tétrahyfro-7,7,9,9-tétraméthyl-5H-benzocyclohepten-2-yl)-propényle];
benzoate d'éthyle p-[(E)-2-(7-éthyl-6,7,8,9-tétrahydro-7-méthyl-5H-benzocyclohepten-2-yl)-propényle];
acide p-[(E)-2-(6,7,8,9-tétrahydro-5H-benzocyclohepten-2-yl)-propényl]benzoïque;
acide p-[(E)-2-(6,7,8,9-tétrahydro-7,7-diméthyl-5H-benzocyclohepten-2-yl)propényl]benzoïque;
acide p-[(E)-2-(6,7,8,9-tétrahydro-5,5,9,9-tétraméthyl-5H-benzocyclohepten-2-yl)propényl]benzoïque;
benzoate d'éthyle p-[(E)-2-(6,7,8,9-tétrahydro-7-méthyl-5H-benzocyclohepten-2-yl)propényle];
acide p-[(E)-2-(6,7,8,9-tétrahydro-6,6,8,8-tétraméthyl-5H-benzocyclohepten-2-yl)propényl]benzoïque;
benzoate de méthyle p-[(E)-(6,7,8,9-tétrahydro-3,7,7-triméthyl-5H-benzocyclohepten-2-yl)-propényle];
benzoate d'éthyle p-[(E)-2-(6,7,8,9-tétrahydro-9-méthyl-5H-benzocyclohepten-2-yl)propényle];
acide p-[(E)-2-(6,7,8,9-tétrahydro-7,7,9,9-tétraméthyl-5H-benzocyclohepten-2-yl)propényl]benzoïque;
acide p-[(Z)-(6,7,8,9-tétrahydro-7,7-diméthyl-3-octyl-5H-benzocyclohepten-2-yl)propényl]benzoïque;
acide p-[(Z)-2-(3-bromo-6,7,8,9-tétrahydro-7,7-diméthyl-5H-benzocyclohepten-2-yl)propényl]-benzoïque;
acide p-[(Z)-2-(6,7,8,9-tétrahydro-3,7,7-triméthyl-5H-benzocyclohepten-2-yl)propényl]benzoïque et
benzoate d'éthyle p-[(E)-2-(6,7,8,9-tétrahydro-5H-benzocyclohepten-2-yl)propényle].

12. Procédé selon la revendication 1 pour la préparation des composés
benzoate d'éthyle p-[(E)-2-[5,6,8,9-tétrahydrospiro[7H-benzocycloheptène-7,1'-cyclopropan]-2-yl]-propényle] et
benzoate d'éthyle p-[(E)-2-[5,6,8,9-tétrahydrospiro[7H-benzocycloheptène-7,1'-cyclopentan]-2-yl]-propényle].

13. Procédé selon la revendication 1 pour la préparation des composés
benzoate d'éthyle p-[(E)-2-(6,7,8,9-tétrahydro-7-oxo-5H-benzocyclohepten-2-yl)propényle] et
benzoate d'éthyle p-[(E)-2-(6,7,8,9-tétrahydro-6,6,8,8-tétraméthyl-7-oxo-5H-benzocyclohepten-2-yl)-propényle].

14. Procédé selon la revendication 1 pour la préparation des composés
ester éthylique de l'acide p-[(E)-2-(6,7,8,9-tétrahydro-10-acétoxy-5,9-diméthyl-5,9-méthano-5H-benzocyclohepten-2-yl)propényl]benzoïque;
acide p-[(E)-2-(6,7,8,9-tétrahydro-10-hydroxy-5,9-diméthyl-5,9-méthano-5H-benzocyclohepten-2-yl)-propényl]benzoïque;

ester éthylique de l'acide p-[(E)-2-(6,7,8,9-tétrahydro-5,9-diméthyl-5,9-méthano-5H-benzocycloheptén-2-yl)propényl]benzoïque et

acide p-[(E)-2-(6,7,8,9-tétrahydro-5,9-diméthyl-5,9-méthano-5H-benzocycloheptén-2-yl)propényl]-benzoïque.

**15.** Procédé pour l'obtention de préparations pharmaceutiques contenant un composé de formule I ou un sel physiologiquement compatible d'un acide carboxylique de formule I, caractérisé en ce que l'on met sous une forme d'utilisation pharmaceutique un tel composé avec des adjuvants et des supports usuels.

**16.** Les composés de formule I ou les sels physiologiquement compatibles des acides carboxyliques de formule I destinés à être utilisés comme substances actives dans des préparations pharmaceutiques présentant une activité anti-séborrhéique, anti-kératinisante, anti-néoplastique, anti-allergique et anti-inflammatoire.